(19) Europäisches Patentamt
European Patent Office
Office européen des brevets

(11) Veröffentlichungsnummer: **0 016 374**
**B1**

(12) # EUROPÄISCHE PATENTSCHRIFT

(45) Veröffentlichungstag der Patentschrift: **03.03.82**

(51) Int. Cl.³: **C 07 D 499/70**, A 61 K 31/43 // C07D239/48, C07D409/12

(21) Anmeldenummer: **80101107.3**

(22) Anmeldetag: **05.03.80**

(54) **Neue Penicilline, ihre Salze, Verfahren zu ihrer Herstellung und diese Verbindungen enthaltende Arzneimittel.**

(30) Priorität: **15.03.79 DE 2910190**

(43) Veröffentlichungstag der Anmeldung: **01.10.80 Patentblatt 80/20**

(45) Bekanntmachung des Hinweises auf die Patenterteilung: **03.03.82 Patentblatt 82/9**

(84) Benannte Vertragsstaaten: **AT BE CH DE FR GB IT LU NL SE**

(56) Entgegenhaltungen: **EP-A-0003814**

(73) Patentinhaber: **Dr. Karl Thomae GmbH, Postfach 720, D-7950 Biberach (Riss) (DE)**

(72) Erfinder: **Wetzel, Bernd, Dr. Dipl.-Chem., Kapellenweg 21, D-7950 Biberach 1 (DE)**
Erfinder: **Woitun, Eberhard, Dr. Dipl.-Chem., Stecherweg 17, D-7950 Biberach 1 (DE)**
Erfinder: **Reuter, Wolfgang, Dr. Dipl.-Chem., Nelkenweg 10, D-7951 Laupertshausen (DE)**
Erfinder: **Maier, Roland, Dr. Dipl.-Chem., Bodelschwinghstrasse 39, D-7950 Biberach 1 (DE)**
Erfinder: **Lechner, Uwe, Dr., Panoramastrasse 12, D-7951 Ummendorf (DE)**
Erfinder: **Goeth, Hanns, Dr., Oberer Bühl 6, D-7950 Biberach 1 (DE)**

EP 0 016 374 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

Neue Penicilline, ihre Salze, Verfahren zu ihrer Herstellung und diese Verbindungen enthaltende Arzneimittel

Die Erfindung betrifft neue Penicilline der allgemeinen Formel I:

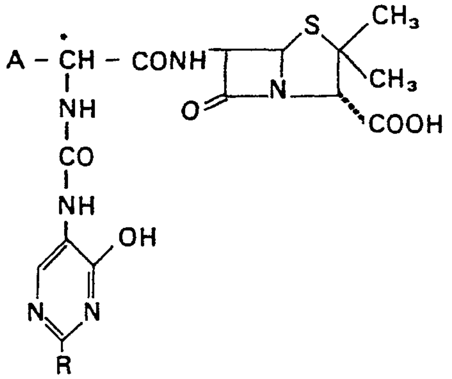

(I)

bzw. deren Tautomere der allgemeinen Formel I':

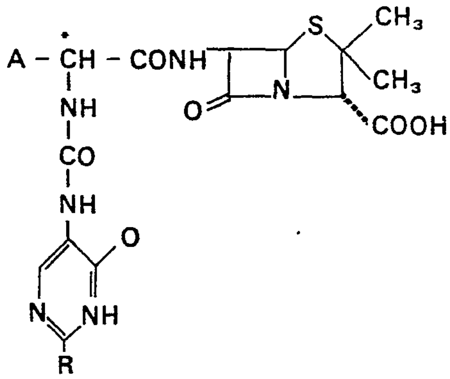

(I')

ihre physiologisch verträglichen Salze mit anorganischen oder organischen Basen, Verfahren zur Herstellung dieser Verbindungen und diese Verbindungen enthaltende Arzneimittel.

In den obigen allgemeinen Formeln I und I' bedeutet:

A die Phenyl-, 4-Hydroxyphenyl- oder die 2- oder 3-Thienylgruppe sowie einen in 3,4-Stellung disubstituierten Phenylrest, wobei die Substituenten gleich oder verschieden und Chloratome, Hydroxy- oder Methoxygruppen sein können,

R eine Gruppe der allgemeinen Formel:

$$NH-Z-X,$$

in der

Z eine geradkettige oder verzweigte Alkylengruppe mit 1 bis 6 Kohlenstoffatomen oder eine durch den Substituenten X substituierte Cycloalkylgruppe mit 3 bis 6 Kohlenstoffatomen bedeutet und

X die Cyanogruppe, die Hydroxygruppe, die Mercaptogruppe, die Aminocarbonyl- oder die Aminosulfonylgruppe oder eine Gruppe der allgemeinen Formeln:

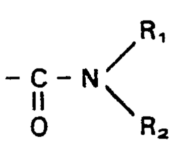 , $-COR_1$, $-COOR_1$, 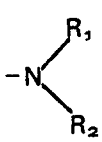 ,

$-NHCOR_1$, 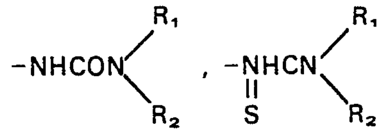 ,

$-NHSO_2R_1$, $-OR_1$, $-OCOR_1$, $-SR_1$, $-SOR_1$, $-SO_2R_1$

sein kann, wobei in diesen Formeln.

$R_1$ eine geradkettige oder verzweigte Alkylgruppe mit 1 bis 4 Kohlenstoffatomen oder die Phenylgruppe und $R_2$ ein Wasserstoffatom oder eine Alkylgruppe mit 1 bis 4 Kohlenstoffatomen darstellt, oder $R_1$ und $R_2$ auch zusammen mit einem eventuell dazwischenliegenden Stickstoffatom einen 3- bis 6gliedrigen monocyclischen heterocyclischen Ring bilden können.

Besonders bevorzugt sind diejenigen Verbindungen der allgemeinen Formeln I bzw. I', in der A die Phenyl- oder p-Hydroxyphenylgruppe darstellt,

$$-\underset{H}{\underset{|}{N}}-Z-$$

eine Gruppe der Formeln

$$-\underset{H}{\underset{|}{N}}-CH_2-CH_2-,\ -\underset{H}{\underset{|}{N}}(CH_2)_3-,$$

$$-\underset{H}{\underset{|}{N}}-\underset{CH_3}{\underset{|}{CH}}-CH_2,\ -\underset{H}{\underset{|}{N}}-CH_2-\underset{CH_3}{\underset{|}{CH}}-$$

$$\text{oder } -\underset{H}{\underset{|}{N}}-CH_2-\overset{CH_3}{\overset{|}{\underset{CH_3}{\underset{|}{C}}}}- \quad \text{bedeutet}$$

und X die Hydroxy-, Methoxy-, Äthoxy-, Methoxycarbonyl-, Äthoxycarbonyl-, Methylcarbonyl-, Äthylcarbonyl-, Methylmercapto-, Äthylmercapto-, Aminocarbonyl-, Aminosulfonyl-, Acetylamino-, Methylsulfinylgruppe oder die Äthylsulfinylgruppe bedeutet, oder in der

$$\underset{H}{\underset{|}{N}}-Z-X$$

die 4'-Hydroxycyclohexylaminogruppe bedeutet.

Die Verbindungen der allgemeinen Formeln I bzw. I' können bezüglich des Chiralitätszentrums C+ in den beiden möglichen R- und S-Konfigurationen vorliegen, jedoch auch als ein Gemisch dieser beiden Konfigurationen. Besonders bevorzugt sind solche Verbindungen, für welche die D = R-Konfiguration zutrifft.

Die Verbindungen der allgemeinen Formeln I bzw. I' zeigen ein breites Wirkungsspektrum gegen grampositive und gramnegative Bakterien bei einer sehr guten Verträglichkeit beim Menschen.

Die Verbindungen der allgemeinen Formeln I bzw. I' lassen sich wie folgt darstellen:

1) Durch Umsetzung einer Verbindung der allgemeinen Formel III:

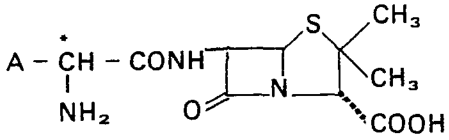 (III)

in der A wie oben definiert ist, mit einem Pyrimidinderivat der allgemeinen Formel IV:

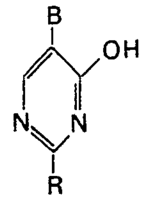 (IV)

in der R wie oben definiert ist und B die Gruppe $-NCO$ oder ein reaktives Derivat der Gruppe NHCOOH bedeutet, wie z.B.

die Gruppen $-NHCOCl$, $-NHCOBr$

oder $- NH - COO -$ 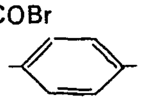 $- NO_2$,

wobei die Gruppe NHCOCl besonders bevorzugt ist. Es können auch Gemische von solchen Pyrimidinderivaten der allgemeinen Formel IV verwendet werden, in der B teils die eine und teils die andere der vorstehend genannten Bedeutungen besitzt, z.B. die Gruppen $-NCO$ und

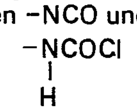

gleichzeitig nebeneinander.

Die Ausgangsprodukte der allgemeinen Formel III können in Form ihrer anorganischen oder organischen Salze, z.B. als Triäthylammoniumsalz oder Natriumsalz, eingesetzt werden. Die Reaktion kann dann in beliebigen Mischungen von Wasser mit solchen organischen Lösungsmitteln, die mit Wasser mischbar sind, wie Ketonen, z.B. Aceton, cyclische Äther, z.B. Tetrahydrofuran oder Dioxan, Nitrilen, z.B. Acetonitril, Formamiden, z.B. Dimethylformamid, Dimethylsulfoxid oder Alkoholen z.B. Isopropanol oder in Hexametapol durchgeführt werden. Dabei hält man den pH der Reaktionsmischung durch Zusatz von Basen oder Verwendung von Pufferlösungen in einem pH-Bereich von etwa 2,0 bis 9,0, vorzugsweise zwischen pH 6,5 und 8,0. Es ist aber auch möglich, die Reaktion in wasserfreien organischen Lösungsmitteln, z.B. halogenierten Kohlenwasserstoffen, wie Chloroform oder Methylenchlorid unter Zusatz von Basen, vorzugsweise Triäthylamin, Diäthylamin oder N-Äthylpiperidin durchzuführen. Weiterhin lässt sich die Reaktion in einem Gemenge aus Wasser und einem mit Wasser nicht mischbaren Lösungsmittel, wie Äther, z.B. Diäthyläther, halogenierten Kohlenwasserstoffen, z.B. Chloroform oder Methylenchlorid, Schwefelkohlenstoff, Ketonen, z.B. Isobutylmethylketon, Estern, z.B. Essigsäureäthylester, aromatischen Lösungsmitteln, z.B. Benzol ausführen, wobei es zweckmässig ist, kräftig zu rühren, und den pH-Wert durch Basenzusatz oder Verwendung von Pufferlösungen in einem Bereich von etwa pH 2,0 bis 9,0, vorzugsweise zwischen 6,5 und 8,0, zu halten. Man kann die Reaktion aber auch in Wasser allein in Gegenwart einer organischen oder anorganischen Base oder unter Zusatz von Pufferstoffen durchführen.

Verwendet man als Ausgangsprodukte für das erfindungsgemässe Verfahren die Silylderivate der Verbindungen der allgemeinen Formel III (z.B. Mono- oder Ditrimethylsilylderivate) und setzt sie mit Verbindungen der allgemeinen Formel IV um, so arbeitet man im allgemeinen zweckmässigerweise in wasser- und hydroxylgruppenfreien Lösungsmitteln, beispielsweise in halogenierten Kohlenwasserstoffen, z.B. Methylenchlorid oder Chloroform, Benzol, Tetrahydrofuran, Aceton oder Dimethylformamid usw. Der Zusatz von Basen ist hierbei nicht notwendig, kann jedoch in einzelnen Fällen vorteilhaft sein, um die Ausbeute und Reinheit der Produkte zu verbessern. Als gegebenenfalls zugesetzte Basen werden zweckmässigerweise tertiäre aliphatische oder aromatische Amine, wie Pyridin oder Triäthylamin, oder durch sterische Hinderung schweracylierbare sekundäre Amine, wie Dicyclohexylamin, benützt.

Statt der Silylester können auch alle anderen Carboxylderivate von α-Aminobenzylpenicillinen, die auf dem Gebiet der Herstellung halbsynthetischer Penicilline bekannt sind, verwendet werden. Typische Beispiele sind die Tritylester, die p-Nitrobenzylester oder die Phenacylester. Im Anschluss an die Umsetzungen können diese Derivate nach bekannten Methoden in die erfindungsgemässen Penicilline umgewandelt werden. Die Menge der verwendeten Basen ist z.B. durch die gewünschte Einhaltung eines bestimmten pH-Wertes festgelegt. Wo eine pH-Messung und Einstellung nicht erfolgt oder wegen des Fehlens von ausreichenden Mengen Wasser im Verdünnungsmittel nicht möglich oder nicht sinnvoll ist, werden im Falle der Verwendung der nichtsilylierten Verbindungen der allgemeinen Formel II vorzugsweise 1,0 bis 2,0 Moläquivalente Basen eingesetzt. Im Falle der Verwendung der silylierten Verbindungen verwendet man vorzugsweise bis zu einem Moläquivalent Base.

Als Basen können prinzipiell alle in der organischen Chemie üblicherweise verwendeten organischen und anorganischen Basen verwendet werden, wie Alkali- und Erdalkalihydroxide, Erdalkalioxide, Alkali- und Erdalkalicarbonate und Hydrogencarbonate, Ammoniak, primäre, sekundäre und tertiäre aliphatische und aromatische Amine sowie heterocyclische Basen. Beispielhaft seien Natrium-, Kalium- und Calciumhydroxid, Calciumoxid, Natrium- und Kaliumcarbonat, Natrium- und Kaliumhydrogencarbonat, Äthylamin, Methyläthylamin, Triäthylamin, Hydroxyäthylamin, Anilin, Pyridin und Piperidin genannt. Bei Verwendung der silylierten Ausgangsstoffe sollten jedoch die oben angegebenen Einschränkungen bezüglich der Art der Basen beachtet werden.

Als Puffersysteme können alle üblichen Puffermischungen verwendet werden, z.B. Phosphatpuffer, Citratpuffer und Tris(hydroxymethyl)aminomethanpuffer.

Die Reaktionstemperaturen können in einem grösseren Bereich variiert werden. Im allgemeinen arbeitet man zwischen etwa −20 und etwa +50° C, vorzugsweise zwischen 0 und +20° C. Die Reaktionspartner der allgemeinen Formeln II und III können von vornherein in äquimolaren Mengen miteinander zur Reaktion gebracht werden. Es kann aber in einzelnen Fällen durchaus zweckmässig sein, einen der beiden Reaktionspartner im Überschuss zu verwenden, um sich damit die Reinigung des Endproduktes zu erleichtern oder um die Ausbeute zu steigern.

2) Durch Umsetzung von Ureidocarbonsäuren der allgemeinen Formel V,

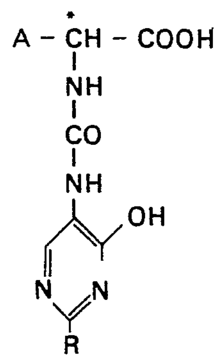

(V)

in der A und R die oben angegebenen Bedeutungen besitzen, ihren Salzen oder reaktiven Derivaten, mit der 6-Aminopenicillansäure der Formel VI,

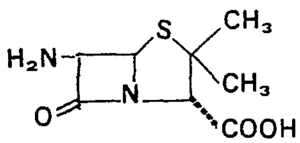

(VI)

oder ihren anorganischen oder organischen Salzen oder Derivaten, die in die 6-Aminopenicillansäure leicht überführbar sind. Das dabei entstehende Reaktionsprodukt wird gegebenenfalls anschliessend zu einem Penicillin der allgemeinen Formel I bzw. I' hydrolysiert oder katalytisch hydrogenolisiert.

Als reaktive Derivate der Ureidocarbonsäuren der allgemeinen Formel V kommen beispielsweise deren Säureanhydride wie z.B. die, die sich von Chlorameisensäureestern, z.B. Chlorameisensäureäthyl- oder -isobutylester, ableiten, oder deren reaktive Ester, wie der p-Nitrophenylester oder der N-Hydroxysuccinimidester, oder deren reaktive Amide, wie das N-Carbonylimidazol, aber auch deren Säurehalogenide, wie das entsprechende Säurechlorid, oder deren Säureazide in Frage. Prinzipiell können jedoch alle Verknüpfungsmethoden, wie sie aus der β-Lactamchemie bekannt sind, verwendet werden.

Die 6-Aminopenicillansäure wird vorteilhafterweise in Form eines ihrer Derivate eingesetzt. Als Derivate kommen hierfür z.B. in Frage: ihr Trimethylsilylester, Tritylester, p-Nitrobenzylester, Phenacylester und ihr O,N-bis-Trimethylsilylderivat. Diese Derivate werden bevorzugt in einem aprotischen Lösungsmittel, wie Methylenchlorid oder Tetrahydrofuran, umgesetzt. Man kann aber auch die 6-Aminopenicillansäure in Form ihrer Salze, beispielsweise ihres Triäthylammoniumsalzes, zum Einsatz bringen; hierbei benutzt man z.B. Methylenchlorid oder ein protisches Lösungsmittel oder ein wässriges Medium oder ein wässrig-organisches Lösungsmittel, wie z.B. Tetrahydrofuran/Wasser-Gemische.

Die Ureidocarbonsäure, ihre Salze oder ihre reaktiven Derivate werden mit der 6-Aminopenicillansäure oder ihren Derivaten in einem Lösungsmittel bei Temperaturen zwischen −40° C und +40° C, gegebenenfalls in Gegenwart einer Base, umgesetzt. Wird z.B. ein Anhydrid der Ureidocarbonsäure, beispielsweise das Anhydrid mit dem Äthylchloroformiat, mit einem Derivat der 6-Aminopenicillansäure umgesetzt, so wird die Reaktion unter Kühlung, beispielsweise bei −10° C bis +10° C in Gegenwart eines tertiären Amins, wie Triäthylamin oder N,N-Dimethylanilin, in einem Lösungsmittel, wie Aceton, Tetrahydrofuran, Dimethylformamid, Chloroform, Dichlormethan, Hexametapol oder in einem Gemisch dieser Lösungsmittel, durchgeführt. Setzt man beispielsweise einen N-Hydroxysuccinimidester der Ureidocarbonsäure mit der 6-Aminopenicillansäure um, so wird die Reaktion vorzugsweise bei 0 bis 20° C in Anwesenheit einer Base, wie z.B. Triäthylamin, in einem Lösungsmittel wie Dimethylformamid, Dichlormethan, Dioxan oder in einem Gemisch solcher Lösungsmittel durchgeführt.

Die Umsetzung einer Ureidocarbonsäure der allgemeinen Formel IV selbst oder ihrer Salze mit der 6-Aminopenicillansäure oder mit deren Salzen erfolgt vorteilhafterweise in Gegenwart eines Kondensationsmittels, z.B. in Gegenwart von N,N'-Dicyclohexylcarbodiimid.

Wird ein Derivat der 6-Aminopenicillansäure eingesetzt, beispielsweise einer ihrer obengenannten Ester, so wird je nach den Reaktionsbedingungen gegebenenfalls ein Reaktionsprodukt erhalten, welches z.B. noch die Esterfunktion enthält. Ein solches Reaktionsprodukt ist aber leicht in das Penicillin der allgemeinen Formel I überführbar. Liegt zum Beispiel die Carboxylgruppe der 6-Aminopenicillansäure in Form ihres Silylesters vor, so kann sie nach der Reaktion in dem erhaltenen Penicillin der allgemeinen Formel I ebenfalls in Form ihres Silylesters vorliegen. In diesem Fall wird anschliessend an die eigentliche Umsetzung diese Silylestergruppe abhydrolysiert, wodurch die Verbindung der allgemeinen Formel I bzw. I' entsteht. In anderen Fällen, z.B. bei Vorliegen eines p-Nitrobenzylesters, wird nach der eigentlichen Umsetzung diese p-Nitrobenzylestergruppe hydrogenolytisch gespalten, wobei dann das Penicillin der allgemeinen Formel I bzw. I' erhalten wird.

Die Aufarbeitung des nach beiden Verfahren erhaltenen Reaktionsgemisches nach erfolgter Umsetzung wird nach den bei β-Lactamantibiotika gebräuchlichen Methoden vorgenommen; dasselbe gilt für die Isolierung und Reinigung der Endprodukte, beispielsweise für die Freisetzung der Säure aus ihren Salzen und die Überführung der freien Säure in andere Salze mittels anorganischer oder organischer Basen. Für die Herstellung der Kalium- oder Natriumsalze bewährt sich besonders die Fällung dieser Salze aus einer alkoholisch-ätherischen Lösung der freien Säure durch die Zugabe von Kalium- oder Natrium-2-äthylhexanoat.

Die als Ausgangstoffe verwendeten Verbindungen der allgemeinen Formel III sind literaturbekannt, vgl. z.B. E.H. Flynn, «Cephalosporines and Penicillines», Academic Press, New York and London, 1972.

Die Ausgangstoffe der allgemeinen Formel IV können beispielsweise durch Umsetzung der entsprechenden 5-Aminopyrimidine der allgemeinen Formel VII:

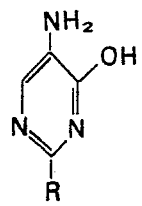

(VII)

in der R wie oben definiert ist, mit Phosgen gewonnen werden. Diese Umsetzung erfolgt vorzugsweise in einem nicht Hydroxylgruppen enthaltenden Lösungsmittel, wie Tetrahydrofuran, Methylenchlorid, Chloroform, Dimethoxyäthan oder Hexametapol bei Temperaturen zwischen −40° und +60°C, vorzugsweise zwischen −10° und +20°C. Dabei empfiehlt es sich, den entstehenden Chlorwasserstoff durch äquimolare Mengen einer inerten organischen Base, wie Triäthylamin oder Pyridin, zu binden. Als Lösungsmittel kann auch Pyridin im Überschuss verwendet werden. Sollten die betreffenden Aminopyrimidine der allgemeinen Formel VII in einem der erwähnten Lösungsmittel schwer löslich sein, kann die Phosgenierung auch in heterogener Phase durchgeführt werden. Des weiteren können die Aminopyrimidine der allgemeinen Formel VII durch Behandlung mit einem Silylierungsmittel, wie Hexamethyldisilazan, Trimethylchlorsilan/Triäthylamin oder Trimethylsilyldiäthylamin, in ein im allgemeinen in den erwähnten Lösungsmitteln sehr leicht lösliches, einfach oder, entsprechend den vorhandenen austauschbaren Wasserstoffatomen, mehrfach silyliertes Aminopyrimidin überführt werden, das mit Phosgen dann zu den entsprechenden Verbindungen der allgemeinen Formel IV reagiert. Je nach der Art des Lösungsmittels, der Höhe der Temperatur, der Menge und Art der eingesetzten Base entsteht entweder überwiegend das entsprechende Isocyanat oder Carbaminsäurehalogenid oder ein Gemisch dieser beiden Verbindungen. Je nach den Reaktionsbedingungen kann die Verbindung der allgemeinen Formel IV auch gering oder teilweise als ein, den Isocyanaten isomeres Tetrahydrooxazolopyrimidin der allgemeinen Formel IVa:

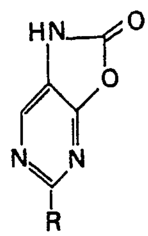

(IVa)

vorliegen.

Die durch Phosgenierung entstandenen Ausgangsprodukte der allgemeinen Formel IV bzw. deren Gemische sind in den obenerwähnten Lösungsmitteln im allgemeinen gut löslich und können, nach Entfernung des überschüssigen Phosgens, ohne weitere Reinigung mit den entsprechenden Penicillinderivaten der allgemeinen Formel III direkt umgesetzt werden.

Die 5-Amino-4-hydroxy-2-subst.-pyrimidine der allgemeinen Formel VII können z.B. nach folgendem Verfahren hergestellt werden:

Durch Umsetzung des 2-Methylmercapto-4-hydroxy-5-nitropyrimidins der nachfolgenden Formel VIII (vgl. Vorbrüggen und Strehlke, «Chem. Ber.», 106, S. 3039, 1973) mit substituierten Alkylaminen der allgemeinen Formel IX, bei der X und Z wie oben definiert sind und anschliessender Reduktion der Nitrogruppe der erhaltenen Verbindung der allgemeinen Formel X nach bekannten Verfahren entsprechend folgendem Reaktionsschema:

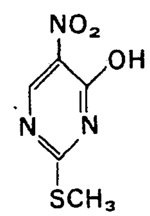

(VIII)

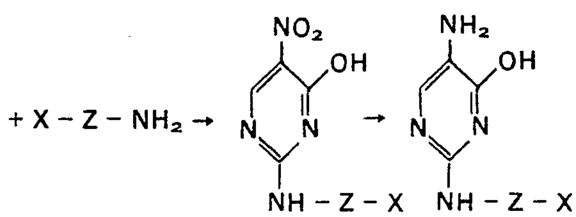


(IX) (X) (VII)

Zur Charakterisierung der Ausgangsprodukte sollen hier einige Vertreter der eingesetzten Pyrimidine der allgemeinen Formel VII genannt werden:

5-Amino-4-hydroxy-2-(2′-hydroxyäthylamino)pyrimidin
5-Amino-4-hydroxy-2-(3′-hydroxypropylamino)pyrimidin
5-Amino-4-hydroxy-2-(2′-hydroxypropylamino)pyrimidin
5-Amino-4-hydroxy-2-(2′-hydroxy-2′-methylpropylamino)pyrimidin

5-Amino-4-hydroxy-2-(2'-methoxyäthylamino)pyrimidin
5-Amino-4-hydroxy-2-(2'-äthoxyäthylamino)pyrimidin
5-Amino-4-hydroxy-2-(3'-methoxypropylamino)pyrimidin
5-Amino-4-hydroxy-2-(äthoxycarbonylmethylamino)pyrimidin
5-Amino-4-hydroxy-(2'-äthoxycarbonyläthylamino)pyrimidin
5-Amino-4-hydroxy-(2'-äthylmercaptoäthylamino)pyrimidin
5-Amino-4-hydroxy-(2'-methylmercaptoäthylamino)pyrimidin
5-Amino-4-hydroxy-(2'-cyanopropylamino)pyrimidin
5-Amino-4-hydroxy-(4'-hydroxycyclohexylamino)pyrimidin
5-Amino-4-hydroxy-(2'-methylcarbonyläthylamino)pyrimidin
5-Amino-4-hydroxy-(3'-aminocarbonylpropylamino)pyrimidin
5-Amino-4-hydroxy-(3'-aminosulfonylpropylamino)pyrimidin
5-Amino-4-hydroxy-(2'-acetylaminoäthylamino)pyrimidin
5-Amino-4-hydroxy-(3'-methylsulfinylpropylamino)pyrimidin

Die Ureidocarbonsäuren der allgemeinen Formel V lassen sich durch Umsetzung der Pyrimidinderivate der allgemeinen Formel IV mit Glycinderivaten der allgemeinen Formel XI:

$$A - \underset{NH_2}{\overset{+}{C}} - COOH \qquad (XI)$$

in der A wie oben definiert ist, erhalten. Die Umsetzung erfolgt bei Temperaturen zwischen -20°C und +40°C, vorzugsweise zwischen 0°C und +20°C, in einem Lösungsmittel. Als Lösungsmittel können hierbei z. B. Gemische von Wasser und organischen Lösungsmitteln, die mit Wasser mischbar sind, verwendet werden, beispielsweise Aceton, Tetrahydrofuran, Dioxan, Acetonitril, Dimethylformamid, Äthanol, Dimethylsulfoxid. Gegebenenfalls wird die Verwendung eines halogenwasserstoffbindenden Mittels notwendig, als solche eignen sich beispielsweise Trialkylamine, wie Triäthylamin, oder anorganische Basen, wie verdünnte Natronlauge.

Es wurde gefunden, daß die Verbindungen der allgemeinen Formel 1 bzw. 1' wertvolle pharmakologische Eigenschaften bei guter Verträglichkeit besitzen. Die erfindungsgemäßen Wirkstoffe können daher zur Prophylaxe und Chemotherapie von lokalen und systemischen Infektionen in der Human- und Tiermedizin verwendet werden. Als Krankheiten, die durch die erfindungsgemäßen Verbindungen verhindert bzw. geheilt werden können, seien beispielsweise solche der Atmungswege, des Rachenraumes und der Harnwege genannt; die Verbindungen wirken insbesondere gegen Pharyngitis, Pneumonie, Peritonitis, Pyelonephritis, Otitis, Cystitis, Endocarditis, Bronchitis, Arthritis und allgemeine systemische Infektionen. Weiter könne diese Verbindungen als Stoffe zur Konservierung von anorganischen und organischen Materialien verwendet werden, besonders von organischen Materialien, wie Polymeren, Schmiermittel, Farben, Fasern, Leder, Papier und Holz sowie von Lebensmitteln.

Dies wird dadurch ermöglicht, daß die Verbindungen der allgemeinen Formel 1 bzw. 1' sowohl *in vitro* als auch *in vivo* gegen schädliche Mikroorganismen, insbesondere gegen grampositive und gramnegative Bakterien sehr stark wirken, wobei sie sich besonders durch ein breites Wirkungsspektrum auszeichnen. Daneben zeigen die Verbindungen der allgemeinen Formel 1 bzw. 1' nach oraler und parenteraler Gabe hohe Spiegel in Gewebe, Serum, Organen und im Urin.

Die folgenden Verbindungen der allgemeinen Formel 1 bzw. 1', in der A und die Gruppe R die in der Tabelle genannten Bedeutungen aufweisen, zeigten eine besonders gute antibakterielle Wirksamkeit:

Tabelle

| A | R |
|---|---|
| Phenyl | $-NHCH_2CH_2OH$ |
| p-Hydroxyphenyl | $-NHCH_2CH_2OH$ |
| p-Hydroxyphenyl | $-NHCH_2C(CH_3)_2OH$ |
| p-Hydroxyphenyl | $-NH(CH_2)_3OH$ |
| Phenyl | $-NH(CH_2)_3OH$ |
| 2-Thienyl | $-NH(CH_2)_3OH$ |
| Phenyl | $-NHCH_2CH(CH_3)OH$ |
| p-Hydroxyphenyl | $-NHCH_2CH(CH_3)OH$ |
| Phenyl | $-NHCH_2CH_2OCH_3$ |
| p-Hydroxyphenyl | $-NHCH_2CH_2OCH_3$ |
| Phenyl | $-NHCH_2CH_2OC_2H_5$ |
| p-Hydroxyphenyl | $-NHCH_2CH_2OC_2H_5$ |
| Phenyl | $-NH(CH_2)_3OCH_3$ |
| p-Hydroxyphenyl | $NH(CH_2)_3OCH_3$ |
| p-Hydroxyphenyl | $NHCH_2COOC_2H_5$ |
| p-Hydroxyphenyl | $NHCH_2CH_2COOC_2H_5$ |
| Phenyl | $NHCH_2CH_2SC_2H_5$ |
| p-Hydroxyphenyl | $NHCH_2CH_2SC_2H_5$ |
| p-Hydroxyphenyl | $NHCH_2CH_2SCH_3$ |
| p-Hydroxyphenyl | $NHCH_2CH(CH_3)-CN$ |
| p-Hydroxyphenyl | $NHCH_2CH_2NHCOCH_3$ |
| p-Hydroxyphenyl | $NHCH_2CH_2CH_2SOCH_3$ |
| p-Hydroxyphenyl | $NHCH_2CH_2CH_2CONH_2$ |
| p-Hydroxyphenyl | $NHCH_2CH_2CH_2SO_2NH_2$ |
| Phenyl | 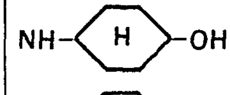 |
| p-Hydroxyphenyl | 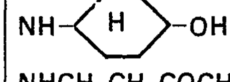 |
| p-Hydroxyphenyl | $NHCH_2CH_2COCH_3$ |

Die Wirksamkeit der erfindungsgemäßen Penicilline läßt sich durch folgende Untersuchungen beispielhaft demonstrieren:

1. In vitro- *Versuche*

Für die Untersuchungen wurde die Methode des Reihenverdünnungstestes im Mikrotitersystem angewandt. Die Prüfung der Substanzen auf Bakteriostase erfolgte in flüssigem Medium. Es wurde die Bakteriostasewirkung bei folgenden Konzentrationen untersucht:
80; 40; 20; 10; 5; 2,5; 1,25; 0,6; 0,3; 0,08 und 0,02 μg/ml.

Es wurde ein Nährboden folgender Zusammensetzung benutzt:
10 g Pepton, 8 g Fleischextrakt Oxoid, 3 g Natriumchlorid, 2 g sek. Natriumphosphat werden mit dest. Wasser auf 1000 ml aufgefüllt (pH 7,2 bis 7,4). Lediglich bei der Testung gegen Streptokokken wurde 1% Glucose hinzugefügt. Das Alter der Primärkulturen betrug etwa 20 h. Die Einstellung der Keimsuspension erfolgte am Photometer (nach Eppendorf) (Reagenzglas durchmesser 14 mm, Filter 546 nm) anhand der Trübung einer Bariumsulfat-Vergleichssuspension, die durch eine Bariumsulfat aufschwemmung erzeugt wurde, welche durch die Zugabe von 3,0 ml 1%ige Bariumchloridlösung in 97 ml 1%ige Schwefelsäure entstand. Nach der Einstellung wurden *Streptococcus* Aronson im Verhältnis 1:15 und die übrigen Testkeime im Verhältnis 1:1500 mit einer Kochsalzlösung weiter verdünnt.

16 mg der jeweiligen Substanz wurden in 10 ml-Meßkolben eingewogen und mit dem Lösungsmittel bis zur Marke aufgefüllt. Die weitere Verdünnungsreihe wurde mit destilliertem Wasser oder dem jeweiligen Lösungsmittel hergestellt.

Die Vertiefungen der Mikrotiterplatten wurden mit 0,2 ml Nährmedium, 0,01 ml der entsprechenden Substanzverdünnung und mit einem Tropfen Keimsuspension (0,01 ml) beschickt und 18 bis 20 h bei 37°C bebrütet. Eine Lösungsmittelkontrolle wurde stets mitgeführt.

Die Ablesung wurde makroskopisch vorgenommen, wobei die jeweilige Grenzkonzentration (= niedrigste noch bakteriostatisch wirksame Konzentration) ermittelt wurde.

Als Testorganismen wurden benützt:
*Staphylococcus aureus* SG 511, *Escherichia coli* ATCC 9637 und 11775, *Pseudomonas aeruginosa hamburgensis* ATCC 10145 und *Pseudomonas aeruginosa* Walter, *Serratia marcescens* ATCC 13880, *Klebsielle pneumoniae* ATCC 10031 und 272 sowie *Proteus mirabilis hamburgensis.*

In der folgenden Tabelle 1 sind die ermittelten minimalen Hemmkonzentrationen (MIC) für typische Vertreter der erfindungsgemäßen Verbindungen aufgeführt:
Natriumsalz von Verbindungen der allgemeinen Formel I bzw. I' mit den Bedeutungen von A und R:

| A | R |
|---|---|
| p-Hydroxyphenyl | $NHCH_2CH_2OCH_3$ =A |
| p-Hydroxyphenyl | $NH(CH_2)_3OH$ =B |
| p-Hydroxyphenyl | NH-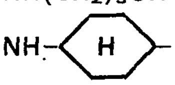-OH =C |
| p-Hydroxyphenyl | $NHCH_2CH_2COOC_2H_5$ =D |
| p-Hydroxyphenyl | $NHCH_2CH_2SC_2H_5$ =E |

Als Vergleichssubstanzen diente das Penicillin der Formel XII (Azlocillin) = F

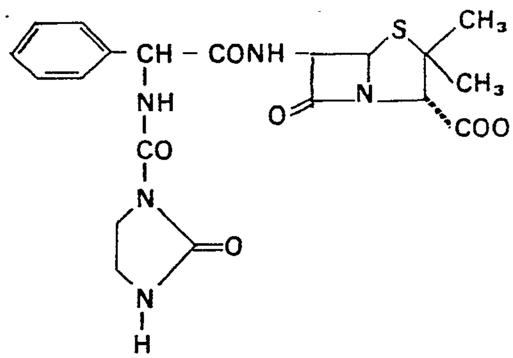
(XII).

Tabelle 1
MHK-Werte verschiedener Penicilline (in μg/ml)

| Verb. | *E. coli* ATCC 9637 | *E. coli* ATCC 11775 | *Pseud. aerug.* Walter | *Pseud. aerug. hamb.* | *Pseud. aerug.* ATCC 10145 | *Serrat. marcesc.* | *Klebs. pneum.* ATCC 10031 | *Klebs. pneum.* 272 | *Prot. mirabilis hamb.* |
|---|---|---|---|---|---|---|---|---|---|
| A | 1,25 | 1,25 | 5 | 1,25 | 1,25 | 0,6 | 20 | 40 | 0,6 |
| B | 0,6 | 0,6 | 5 | 1,25 | 1,25 | 1,25 | 10 | 10 | 0,3 |
| C | 0,6 | 0,6 | 2,5 | 2,5 | 1,25 | 1,25 | 20 | 10 | 0,08 |
| D | 0,3 | 0,6 | 2,5 | 2,5 | 2,5 | 1,25 | 10 | 10 | 0,3 |
| E | 0,6 | 0,6 | 2,5 | 2,5 | 1,25 | 0,6 | 10 | 10 | 0,08 |
| F | 10 | 10 | 10 | 10 | 5 | 5 | 40 | 40 | 1,25 |

Wie aus der vorstehenden Tabelle ersichtlich wird, sind die genannten Verbindungen der Vergleichssubstanz F in ihrer Wirksamkeit gegenüber typischen gramnegativen Hospitalkeimen, deutlich überlegen.

Die Akute Toxizität wurde durch perorale und subcutane Applikation der Verbindungen der Tabelle 1 an weiße Laboratoriumsmäuse in steigenden Dosen bestimmt.

Die $LD_{50}$ ist die Dosis, nach deren Applikation 50% der Tiere innerhalb von 8 d sterben. Sämtliche Substanzen zeigten bei oraler Gabe eine $LD_{50}$ von über 4 g/kg, bei subcutaner Gabe eine $LD_{50}$ von über 3 g/kg, d.h. bei 3g/kg starben keine Tiere, sie sind damit für die Praxis untoxisch.

Eine Reihe der erfindungsgemäßen Verbindungen wurde *in vivo* bei experimentellen Infektionen an Mäusen untersucht. Man verwendete als pathogene Bakterien *E. coli* ATCC 11775 und *Pseudomonas aeruginosa* Walter. Es wurde eine intraperitoneale Infektion mit 0,2 ml einer Bakteriensuspension (mit 5% Mucin) gesetzt. Dies entspricht etwa 1,4 × $10^6$ Keime *E. coli* bzw. 1,3 × $10^6$ Keime *Pseudomonas* Maus. Weibliche Mäuse von Stamm NMRI wurden in Gruppen von jeweils 10 Tieren aufgeteilt, zwei Gruppen blieben unbehandelt, die restlichen Gruppen wurden mit verschiedenen Dosen der jeweiligen erfindungsgemäßen Penicilline subcutan zur Bestimmung der $ED_{50}$ (Dosis bei der 50% der Tiere überleben) behandelt. Bei der *E. coli*-Infektion wurde am ersten Tag dreimal therapiert (1, 4 und 7 h post-Infektionen) und am 2. Tag 2 mal. Bei der *Pseudomonas*-Infektion wurde am ersten Tag 6× (1,3,5,7,9,11 h post-Infektionem) und an den darauffolgenden 2 d zweimal täglich behandelt.

Der Beobachtungszeitraum betrug in beiden Fällen 7 d. Die Ergebnisse dieser Teste mit repräsentativen Vertretern der erfindungsgemäßen Penicilline sind in der Tabelle 2 dargestellt.

Tabelle 2

In vivo *Aktivität bei Mäusen*

a) E. coli- *Infektion (s.c. Applikation):*

| Verbindung | $ED_{50}$ (mg/kg) |
|---|---|
| A | 2 |
| B | 0,8 |
| C | 0,7 |
| D | 4,2 |
| E | 1,7 |
| F | 35 |

b) Pseudomonas *(s.c. Applikation):*

| Verbindung | $ED_{50}$ (mg/kg) |
|---|---|
| A | 1,5 |
| B | 1,2 |
| C | 1,7 |
| E | 2,3 |
| F | 110 |

Auch hierbei zeigt sich wieder eine signifikante Überlegenheit der Substanzen A bis E gegenüber der bekannten Substanz F.

Es ist eine weitere Aufgabe der vorliegenden Erfindung, pharmazeutische Mittel zu schaffen, die bei der Behandlung infektiöser Krankheiten sowohl beim Menschen als auch beim Tier wertvoll sind.

Als bevorzugte pharmazeutische Zubereitungen seien Tabletten, Dragées, Kapseln, Granulate, Suppositorien, Lösungen, Suspensionen, Emulsionen, Salben, Gele, Cremes, Puder und Sprays genannt. Vorteilhafterweise wird der Wirkstoff in der Human- oder Tiermedizin oder ein Gemisch der verschiedenen Wirkstoffe der allgemeinen Formel I bzw. I' in einer Dosierung zwischen 5 und 500, vorzugsweise 10 bis 200 mg/kg Körpergewicht je 24 h verabreicht, gegebenenfalls in Form mehrerer Einzelgaben. Eine Einzelgabe enthält den oder die erfindungsgemäßen Wirkstoffe, vorzugsweise in Mengen von etwa 1 bis etwa 250, insbesondere 10 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzeinmittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive Bakterien verhindert, gebessert und/oder geheilt werden und ebenso eine Förderung des Wachstums und eine Verbesserung der Verwertung des Futters erreicht werden.

Die folgenden Beispiele sollen die Erfindung näher erläutern:

Mit Ampicillin ist dasjenige α-Aminobenzylpenicillin und mit Amoxycillin dasjenige α-Aminop-hydroxybenzylpenicillin mit der D=R-Konfiguration in der Seitenkette gemeint.

## I. Darstellung der Ausgangsprodukte

*Beispiel A*

5-Amino-4-hydroxy-2-(2'-methoxyäthylamino) pyrimidin

a) 5,03 g (0,025 mol) 2-Äthylmercapto-4-hydroxy-5-nitropyrimidin werden in 100 ml Äthanol mit 3,76 g (0,05 mol) 2-Methoxyäthylamin 16 h lang am Rückfluß erhitzt. Nach dem Abkühlen wird das ausgefallenen Produkt abgesaugt, mit wenig Äthanol gewaschen und getrocknet. Die Substanz wird dann mit Wasser verrührt und mit 2N-Salzsäure angesäuert. Anschließend wird abgesaugt, mit Wasser gewaschen und getrocknet.

Ausbeute: 3,9 g (7,3%)
Fp.: 222°C

*Analyse:*

| | | | |
|---|---|---|---|
| berechnet: | C 39,25 | H 4,71 | N 26,16% |
| gefunden: | C 38,76 | H 4,91 | N 26,10% |

b) 3,9 g der so erhaltenen Nitroverbindung werden in 200 ml Äthanol mit 3,9 g Raney-Nickel 2 h lang bei 50°C hydriert. Das nach Abdestillieren des Äthanol zurückbleibende schmierige Produkt wird rasch über eine kurze Silicagelsäule filtriert (Eluens Methylenchlorid/Methanol 3:1)

Das erhaltene Produkt (2,1 g = 63%) zerläuft an der Luft zu einer schmierigen Substanz.

*Analyse:*

| | | | |
|---|---|---|---|
| berechnet: | C 45,64 | H 6,57 | N 30,42% |
| gefunden: | C 44,97 | H 6,88 | N 29,11% |

IR-Spektrum: 3260, 3050 (breit), 2960, 2930, 2880, 1670, 1610, 1580, 1110 $cm^{-1}$.

NMR-Spektrum (DMSO + $CD_3OD$) Signale bei ppm: 3,3 (s,3H), 3,5 (m,4H), 7,05 (s,1H).

Analog wurden folgende Aminopyrimidine der allgemeinen Formel VII synthetisiert:

| R | Ausbeute (%) | | Analyse C | H | N |
|---|---|---|---|---|---|
| $NH(CH_2)_2OC_2H_5$ | 47 | ber.<br>gef. | 48,47<br>47,91 | 7,12<br>7,24 | 28,27<br>27,95 |
| $NH(CH_2)_2OC_3H_7$ | 51 | ber.<br>gef. | 50,93<br>51,01 | 7,60<br>8,27 | 26,40<br>25,95 |
| $NH(CH_2)_2OC_4H_9$ | 41 | ber.<br>gef. | 53,07<br>52,81 | 8,02<br>8,17 | 24,76<br>24,01 |
| $NH(CH_2)_3OCH_3$ | 38 | ber.<br>gef. | 48,47<br>48,07 | 7,12<br>6,99 | 28,27<br>27,75 |
| $NH(CH_2)_3OC_2H_5$ | 52 | ber.<br>gef. | 50,93<br>50,42 | 7,60<br>7,87 | 26,46<br>26,12 |
| $NH(CH_2)_3OC_3H_7$ | 50 | ber.<br>gef. | 53,07<br>53,01 | 8,02<br>8,12 | 24,76<br>24,04 |
| $NH(CH_2)_2OC_6H_5$ | 28 | ber.<br>gef. | 58,52<br>58,43 | 5,73<br>5,87 | 22,75<br>22,50 |
| $NH(CH_2)_2OH$ | 40 | ber.<br>gef. | 42,35<br>41,89 | 5,92<br>5,76 | 32,93<br>31,88 |
| $NH(CH_2)_3OH$ | 37 | ber.<br>gef. | 45,64<br>45,70 | 6,57<br>6,65 | 30,42<br>29,32 |
| $NHCH_2CH(CH_3)OH$ | 54 | ber.<br>gef. | 45,64<br>45,07 | 6,57<br>6,70 | 30,42<br>30,40 |
| $NHCH_2C(CH_3)_2OH$ | 58 | ber.<br>gef. | 48,47<br>48,45 | 7,12<br>7,14 | 28,27<br>28,20 |
| 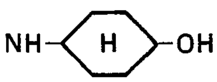 | 54 | ber.<br>gef. | 53,86<br>53,58 | 7,19<br>7,66 | 24,98<br>24,95 |
| $NHCH_2CH_2N(C_2H_5)_2$ | 38 | ber.<br>gef. | 53,31<br>53,78 | 8,50<br>8,72 | 31,09<br>30,60 |
| $NHCH_2COOC_2H_5$ | 46 | ber.<br>gef. | 45,28<br>45,37 | 5,70<br>5,96 | 26,40<br>25,35 |
| $NHCH_2CH_2COOC_2H_5$ | 44,5 | ber.<br>gef. | 47,78<br>46,90 | 6,24<br>6,17 | 24,77<br>23,80 |
| $NHCH_2CH_2SCH_3$ | 31 | ber.<br>gef. | 42,00<br>41,53 | 6,00<br>6,24 | 28,01<br>27,07 |
| $NHCH_2CH_2SC_2H_5$ | 43<br>klebrige<br>Substanz | ber.<br>gef. | 44,84<br>44,75 | 6,59<br>6,71 | 26,15<br>25,75 |
| $NHCH_2CH(CH_3)CN$ | 56 | ber.<br>gef. | 49,74<br>49,22 | 6,00<br>6,17 | 36,27<br>35,64 |
| $NHCH_2CH_2COCH_3$ | 54,5 | ber.<br>gef. | 48,98<br>49,64 | 6,12<br>6,21 | 28,57<br>27,89 |

| R | Ausbeute (%) | | Analyse C | H | N |
|---|---|---|---|---|---|
| $NHCH_2CH_2NHC_6H_5$ | 61,5 | ber. | 58,78 | 6,12 | 28,57 |
| | | gef. | 58,21 | 6,27 | 27,44 |
| $NHCH_2CH_2NHCOCH_3$ | 51 | ber. | 45,49 | 6,20 | 33,16 |
| | | gef. | 45,22 | 6,29 | 32,20 |
| $NH(CH_2)_3CONH_2$ | 31 | ber. | 45,50 | 6,16 | 33,18 |
| | | gef. | 46,01 | 6,42 | 31,94 |
| $NH(CH_2)_3SO_2NH_2$ | 42,5 | ber. | 34,01 | 4,86 | 28,34 |
| | | gef. | 34,44 | 5,04 | 27,80 |

*Beispiel B*

*5-Amino-2-(2'-äthylsulfinyläthylamino)-4-hydroxypyrimidin*

3,2 g (0,015 mol) 5-Amino-2-(2'-äthylmercaptoäthylamino)-4-hydroxypyrimidin werden in einem Gemisch aus 60 ml abs. Methanol und 60 ml abs. Methylenchlorid gelöst und bei −15°C mit 2,89 g (0,015 mol) m-Chlorperbenzoesäure, gelöst in 50 ml Methylenchlorid, versetzt. Es wird 8 h lang bei 0°C gerührt.

Das Gemisch wird im Vakuum fast zur Trockne eingeengt und auf einer Silicagelsäule mit einem Methylenchlorid/Methanolgemisch von zunächst 10:1, dann 5:1 fraktioniert. Man erhält 1,42 g (41,1%) eines einheitlichen Produkts.

*Analyse:*

| | | | |
|---|---|---|---|
| berechnet: | C 41,72 | H 6,13 | N 24,33% |
| gefunden: | C 41,44 | H 6,12 | N 24,48% |

IR-Spektrum: 1650, 1610, 1530, 1010 $cm^{-1}$.

NMR-Spektrum (DMSO + $CD_3OD$) Signale bei ppm: 1,5 (t,3H), 2,8 (m,4H), 3,4 (t,2H), 7,1 (s,1H)

Analog wurde hergestellt: 5-Amino-2-(3'-methylsulfinylpropylamino)-4-hydroxypyrimidin

Ausbeute: 36,5%

*Analyse:*

| | | | |
|---|---|---|---|
| berechnet: | C 41,72 | H 6,13 | N 24,33% |
| gefunden: | C 41,99 | H 6,24 | N 23,60% |

NMR-Spektrum (DMSO + $CD_3OD$) Signale bei ppm: 1,8(m,2H), 2,4 (s,3H), 2,8 (t,2H), 3,4 (t,2H), 7,05 (s,1H)

*Beispiel C*

*D-α-[3-(4-Hydroxy-2-{2'-methoxyäthylamino}-5-pyrimidinyl)ureido]phenylglycin*

1,84 g (0,01 mol) 5-Amino-4-hydroxy-2-(2'-methoxyäthylamino)pyrimidin werden in absolutem Tetrahydrofuran gelöst, mit 1,35 ml Triäthylamin versetzt und unter Eiskühlung zu 1,05 g Phosgen in 30 ml Tetrahydrofuran getropft. Anschließend wird das Gemisch im Vakuum auf etwa 50 ml eingeengt und dann unter Eiskühlung zu einer Lösung von 1,51 g (0,01 mol) D-Phenylglycin, das mit 10 ml 1 N-Natronlauge in einem 1:1 Gemisch Tetrahydrofuran, wassergelöst wurde, getropft. Nach Zusatz rührt man 2 h bei Raumtemperatur nach, wobei der pH-Wert mit N/10-Natronlauge auf etwa 9 gehalten wird.

Anschließend wird das Tetrahydrofuran im Vakuum entfernt. Die wäßrige Lösung wird bei pH 7 zweimal mit je 100 ml Essigsäure äthylester ausgeschüttelt, denn auf pH 3,1 angesäuert und das ausgefallene Produkt abgesaugt. Nach Trocknen über $P_2O_5$ bleiben 2,06 g (57%) farbloses Festprodukt zurück.

IR-Spektrum 1720 (Schulter), 1680 (breit), 1550, 1570 $cm^{-1}$

NMR-Spektrum (DMSO + $CD_3OD$) Signale bei ppm: 3,3 (2H), 3,5 (3H), 3,6 (2H), 5,2 (1H), 7,3 (5H), 8,15 (1H)

*Analyse:*

| | | | |
|---|---|---|---|
| berechnet: | C 53,18 | H 9,90 | N 19,39% |
| gefunden: | C 54,02 | H 10,11 | N 18,78% |

Analog wurden synthetisiert:

D-α-[3-(2-(2'-Äthylmercaptoäthylamino)-4-hydroxy-5-pyrimidinyl)ureido]-p-phenylglycin

*Beispiel D*

*D,L-α-[3-(4-Hydroxy-2-(3'-hydroxypropylamino)-5-pyrimidinyl)ureido]thienylglycin*

1,84 g (0,01 mol) 5-Amino-4-hydroxy-2-(3'-hydroxypropylamino)pyrimidin werden 10 min lang mit 7 ml Diäthylaminotrimethylsilan auf 80°C erwärmt. Die entstandene Lösung wird im Vakuum

zur Trockne eingeengt, und das zurückbleibende Festprodukt in 30 ml absolutem Tetrahydrofuran gelöst. Diese Lösung wird bei 0°C zu einer Lösung von 1,05 g Phosgen in 50 ml trockenem Tetrahydrofuran getropft. Man rührt 15 min bei Raumtemperatur nach. Anschließend wird im Vakuum auf etwa 40 ml eingeengt.

Man löst 1,57 g D,L-Thienylglycin in 40 ml Tetrahydrofuran und 10 ml 1N-Natronlauge. Zu dieser Lösung tropft man unter Eiskühlung die oben erhaltene Lösung zu. Der pH-Wert wird mit 1N-Natronlauge dabei auf etwa 8,0 gehalten. Nach der Zugabe wird 1 h bei 5°C und 1 h bei Raumtemperatur gerührt. Die Aufarbeitung erfolgt analog Beispiel IC.

Ausbeute: 2,55 g (69,5%)

IR-Spektrum: 1720, 1675, 1545, 1470 $cm^{-1}$

NMR-Spektrum (DMSO + $CD_3OD$) Signale bei ppm: 1,8 (m,2H), 3,45 (m,4H), 5,5 (s,1H), 6,9 (breit,2H), 7,25 (d,1H), 8,05 (s,1H)

*Analyse:*

| | | | | |
|---|---|---|---|---|
| berechnet: | C 45,78 | H 4,63 | N 19,07 | S 8,72% |
| gefunden: | C 46,12 | H 4,90 | N 18,77 | S 8,22% |

## II. Darstellung der Endprodukte

*Beispiel 1*

*D-α-[3-(4-Hydroxy-2-{2'-methoxyäthylamino}-5-pyrimidinyl)ureido]-p-hydroxybenzylpenicillinnatrium*

920 mg (0,005 mol) 5-Amino-2-(2'-methoxyäthylamino)-4-hydroxypyrimidin werden unter Erwärmen in 250 ml absolutem Tetrahydrofuran gelöst und mit 500 mg Triäthylamin versetzt. Diese Lösung wird bei 0°C zu einer Lösung von 500 mg Phosgen in 30 ml absolutem Tetrahydrofuran getropft. Man rührt unter Eiskühlung etwa 30 min nach. Anschließend wird Stickstoff durch die Lösung geblasen, um nicht umgesetztes Phosgen zu entfernen.

Man suspendiert 2,1 g (0,005 mol) Amoxicillintrihydrat in 80 ml wäßrigem 80%igem Tetrahydrofuran und kühlt auf 0°C ab. Dann wird soviel Triäthylamin zugegeben, daß eine Lösung entsteht. Innerhalb von 5 min wird die oben hergestellte Suspension zugetropft, wobei der pH-Wert mit Triäthylamin auf 7,5 gehalten wird. Man setzt weitere 30 ml Wasser zu und hält das Reaktionsgemisch 1 h bei 0 bis 2°C. Die Kühlung wird entfernt und es wird 1 h bei Raumtemperatur nachgerührt.

Man setzt dann 40 ml Wasser zu und entfernt das Tetrahydrofuran im Vakuum. Die zurückbleibende Wasserphase wird zweimal mit 50 ml Essigsäureäthylester gewaschen. Unter Rühren wird dann zu der wäßrigen Lösung langsam 2N-Salzsäure zugetropft bis pH 2,9, wobei die Temperatur unter 5°C gehalten wird. Das ausgefallene Produkt wird abgesaugt und im Exsikkator getrocknet. Zu dem Festprodukt wird eine Lösung von 700 mg Natriumhexanoat in 25 ml Methanol gegeben und die entstehende Lösung mit Äther versetzt. Das ausgefallene Natriumsalz wird abgesaugt und im Vakuum getrocknet.

Ausbeute: 2,14 g (71,2%)

IR-Spektrum: 1765, 1660, 1610, 1540 $cm^{-1}$

NMR-Spektrum (DMSO + $CD_3OD$) Signale bei ppm: 1,5 (d,6H), 3,3 (s,3H), 3,5 (m,4H), 4,0 (s,1H), 5,4 (q,2H), 5,5 (s,1H), 6,8 (d,2H), 7,3 (d,2H), 8,15 (s,1H)

*Beispiel 2*

*D-α-[3-(2-{2'-Äthoxyäthylamino}-4-hydroxy-5-pyrimidinyl)ureido]-p-hydroxybenzylpenicillinnatrium*

Dieses Penicillin wurde analog Beispiel 1 hergestellt. Man geht aus von 1,68 g (0,004 mol) Amoxicillintrihydrat sowie dem Umsetzungsprodukt von 790 mg (0,004 mol) 5-Amino-2-(2'-äthoxyäthylamino)-4-hydroxypyrimidin mit 400 mg Phosgen und 0,53 ml Triäthylamin.

Ausbeute: 1,58 g Natriumsalz (64,2%)

IR-Spektrum: 1770, 1660, 1610, 1550, 1510 $cm^{-1}$

NMR-Spektrum (DMSO + $CD_3OD$) Signale bei ppm: 1,15 (t,3H), 1,55 (d,6H), 3,6 (m,6H), 4,05 (s,1H), 5,45 (m,3H), 6,8 (d,2H), 7,3 (d,2H), 8,15 (s,1H).

*Beispiel 3*

*D-α-[3-(2-{2'-Äthoxyäthylamino}-4-hydroxy-5-pyrimidinyl)ureido]benzylpenicillinnatrium*

Dieses Penicillin wurde analog Beispiel 1 hergestellt. Man geht aus von 2,25 g (0,006 mol) Ampicillinnatriumsalz sowie dem Umsetzungsprodukt von 1,19 g (0,006 mol) des Pyrimidins des Beispiels 2 mit 600 mg Phosgen und 0,8 ml Triäthylamin.

Ausbeute: 2,11 g Natriumsalz (59%)

IR-Spektrum: 1765, 1650, 1610, 1545 $cm^{-1}$

NMR-Spektrum (DMSO + $CD_3OD$) Signale bei ppm: 1,15 (t,3H), 1,50 (d,6H), 3,65 (m,6H), 4,05 (s,1H), 5,45 (q,2H), 5,55 (s,1H), 7,4 (m,5H), 8,10 (s,1H)

*Beispiel 4*

*D-α-[3-(4-Hydroxy-2-{2'-propoxyäthylamino}-5-pyrimidinyl)ureido]-p-hydroxybenzylpenicillinnatrium*

Dieses Penicillin wurde analog Beispiel 1 hergestellt. Man geht aus von 2,1 g (0,005 mol) Amoxicillintrihydrat sowie dem Umsetzungsprodukt von 1,13 g 5-Amino-4-hydroxy-2-(2'-propoxyäthylamino)pyrimidin mit 500 mg Phosgen und 0,68 ml Triäthylamin.

Ausbeute: 1,8 g Natriumsalz (57,5%)

IR-Spektrum: 1765, 1650, 1615, 1545 $cm^{-1}$

NMR-Spektrum (DMSO + $CD_3OD$) Signale bei ppm: 1,0 (t,3H), 1,45 (m,2H), 1,55 (d,6H), 3,55 (m,6H), 4,05 (s,1H), 5,45 (q,2H), 5,5 (s,1H), 6,8 (d,2H), 7,3 (d,2H), 8,15 (s,1H).

*Beispiel 5*

*D-α-[3-(2-{2'-Butoxyäthylamino}-4-hydroxy-5-pyrimidinyl)ureido]-p-hydroxybenzylpenicillinnatrium*

Dieses Penicillin wurde analog Beispiel 1 hergestellt. Man geht aus von 840 mg (0,002 mol) Amoxicillintrihydrat sowie dem Umsetzungsprodukt von 450 mg (0,002 mol) 5-Amino-2-(2'-butoxyäthylamino)-4-hydroxypyrimidin mit 200 mg Phosgen und 0,27 ml Triäthylamin.

Ausbeute: 615 mg Natriumsalz (48%)

IR-Spektrum: 1770, 1650, 1615, 1550 $cm^{-1}$

NMR-Spektrum ($DMSO + CD_3OD$) Signale bei ppm: 0,9 (t,3H), 1,4 (m,4H), 1,6 (d,6H), 3,5 (m,6H), 4,05 (s,1H), 5,45 (m,3H), 6,8 (d,2H), 7,35 (d,2H), 8,2 (s,1H).

*Beispiel 6*

*D-α-[3-(4-Hydroxy-2-{3'-methoxypropylamino}-5-pyrimidinyl)ureido]-p-hydroxybenzylpenicillinnatrium*

Dieses Penicillin wurde analog Beispiel 1 hergestellt. Man geht aus von 2,1 g (0,005 mol) Amoxicillintrihydrat sowie dem Umsetzungsprodukt von 1,0 g (0,005 mol) 5-Amino-4-hydroxy-2-(3'-methoxypropylamino)pyrimidin mit 0,5 g Phosgen und 0,68 ml Triäthylamin.

Ausbeute: 1,82 g Natriumsalz (60%)

IR-Spektrum: 1765, 1650, 1600, 1500 $cm^{-1}$

NMR-Spektrum ($DMSO + CD_3OD$) Signale bei ppm: 1,55 (d,6H), 1,8 (m,2H), 3,2 bis 3,6 (m,7H), 4,05 (s,1H), 5,4 (q,2H), 5,5 (s,1H), 6,8 (d,2H), 7,35 (d,2H), 8,15 (s,1H).

*Beispiel 7*

*D-α-[3-(4-Hydroxy-2-{3'-methoxypropylamino}-5-pyrimidinyl)ureido]-benzylpenicillinnatrium*

Dieses Penicillin wurde analog Beispiel 1 hergestellt. Man geht aus von 4,5 g (0,012 mol) Ampicillinnatriumsalz sowie dem Unsetzungsprodukt von 2,4 g (0,012 mol) des Pyrimidins des Beispiels 6 mit 1,25 g Phosgen und 1,65 ml Triäthylamin.

Ausbeute: 4,6 g Natriumsalz (66%)

IR-Spektrum: 1765, 1650, 1610, 1510 $cm^{-1}$

NMR-Spektrum ($DMSO + CD_3OD$) Signale bei ppm: 1,55 (d,6H), 1,85 (m,2H), 3,2 bis 3,65 (m,7H), 4,0 (s,1H), 5,45 (q,2H), 5,6 (s,1H), 7,35 (m,5H), 8,10 (s,1H).

*Beispiel 8*

*D-α-[3-(2-{3'-Äthoxypropylamino}-4-hydroxy-5-pyrimidinyl)ureido]-p-hydroxybenzylpenicillinnatrium*

Dieses Penicillin wurde analog Beispiel 1 hergestellt. Man geht aus von 1,89 g (0,0045 mol) Amoxicillintrihydrat sowie dem Umsetzungsprodukt von 950 mg (0,0045 mol) 5-Amino-2-(β-äthoxypropylamino)-4-hydroxypyrimidin mit 450 mg Phosgen und 450 mg Triäthylamin.

Ausbeute: 1,9 g Natriumsalz (68%)

IR-Spektrum: 1770, 1670, 1610, 1550, 1510 $cm^{-1}$

NMR-Spektrum ($DMSO + CD_3OD$) Signale bei ppm: 1,05 (t,3H), 1,55 (d,6H), 1,7 (m,2H), 3,4 (m,6H), 4,05 (s,1H), 5,45 (q+s,3H), 6,8 (d,2H), 7,35 (d,2H), 8,10 (s,1H).

*Beispiel 9*

*D-α-[3-(4-Hydroxy-2-{3'-isopropoxypropylamino}-5-pyrimidinyl)ureido]-p-hydroxybenzylpenicillinnatrium*

Dieses Penicillin wurde analog Beispiel 1 hergestellt. Man geht aus von 4,2 g (0,01 mol) Amoxicillin trihydrat sowie dem Umsetzungsprodukt von 2,25 g (0,01 mol) 5-Amino-4-hydroxy-2-(3'-isopropoxypropylamino)pyrimidin mit 1,0 g Phosgen und 1,35 ml Triäthylamin.

Ausbeute: 4,67 g Natriumsalz (73%)

IR-Spektrum: 1770, 1665, 1615, 1550 $cm^{-1}$

NMR-Spektrum ($DMSO + CD_3OD$) Signale bei ppm: 1,15 (d,6H), 1,55 (d,6H), 1,80 (m,2H), 3,2 bis 3,8 (m,5H), 4,05 (s,1H), 5,5 (q+s,3H), 6,8 (d,2H), 7,3 (d,2H), 8,15 (s,1H).

*Beispiel 10*

*D-α-[3-(4-Hydroxy-2-{2'-hydroxy-2'-methylpropylamino}-5-pyrimidinyl)-ureido]-p-hydroxybenzylpenicillinnatrium*

Dieses Penicillin wurde analog Beispiel 1 hergestellt. Man geht aus von 1,26 g (0,003 mol) Amoxicillin trihydrat sowie dem Umsetzungsprodukt von 590 mg (0,003 mol) 5-Amino-4-hydroxy-2-(2'-hydroxy-2'-methylpropylamino)pyrimidin mit 300 mg Phosgen und 0,41 ml Triäthylamin.

Ausbeute: 720 mg Natriumsalz (39,5%)

IR-Spektrum: 1765, 1660, 1610, 1550 $cm^{-1}$

NMR-Spektrum ($DMSO + CD_3OD$) Signale bei ppm: 1,2 (6H), 1,55 (d,6H), 3,5 (2H), 4,05 (1H), 5,5 (q+s,3H), 6,85 (d,2H), 7,35 (d,2H), 8,15 (s,1H).

*Beispiel 11*

*D-α-[3-(4-Hydroxy-2-{2'-hydroxyäthylamino}-5-pyrimidinyl)ureido]-p-hydroxybenzylpenicillinnatrium*

510 mg (0,003 mol) 5-Amino-4-hydroxy-2-(2'-hydroxyäthylamino) pyrimidin werden bei 80°C 15 min lang mit 3 ml Trimethylsilyldiäthylamin gerührt. Die entstandene Lösung wird im Wasserstrahlvakuum und anschließend in Hochvakuum zur Trockne eingeengt. Dann nimmt man in 50 ml absoluten Tetrahydrofuran auf. Diese Lösung wird unter Eiskühlung zu einer Lösung von 300 mg Phosgen in 20 ml absoluten Tetrahydrofuran getropft.

Die weitere Umsetzung mit 1,26 g Amoxicillin (0,003 mol) erfolgt wie in den vorhergegangenen Beispielen angegeben.

Ausbeute: 495 mg Natriumsalz (29%)

IR-Spektrum: 1770, 1665, 1615, 1550 $cm^{-1}$

NMR-Spektrum (DMSO + $CD_3OD$) Signale bei ppm: 1,55 (d,6H), 3,5 (m,4H), 4,05 (s,1H), 5,45 (q,2H), 5,50 (s,1H), 6,85 (d,2H), 7,35 (d,2H), 8,10 (s,1H).

*Beispiel 12*

*D-α-[3-(4-Hydroxy-2-{2'-hydroxyäthyl-amino}-5-pyrimidinyl)ureido]-benzylpenicillinnatrium*

Diese Penicillin wurde analog Beispiel 11 synthetisiert. Man geht aus von 1,8 g (0,005 mol) Ampicillinnatriumsalz sowie dem Umsetzungsprodukt von 500 mg des Amins des Beispiels 11 mit 500 mg Phosgen.

Ausbeute: 950 mg Natriumsalz (34%)

IR-Spektrum: 1770, 1660, 1620, 1510 $cm^{-1}$

NMR-Spektrum (DMSO + $CD_3OD$) Signale bei ppm: 1,50 (d,6H), 3,2 bis 3,7 (m,4H), 4,0 (s,1H), 5,45 (q,2H), 5,60 (s,1H), 7,4 (m,5H), 8,10 (s,1H).

*Beispiel 13*

*D-α-[3-(4-Hydroxy-2-{3'-hydroxypropyl-amino}-5-pyrimidinyl)ureido]-p-hydroxybenzylpenicillinnatrium*

Dieses Penicillin wurde analog Beispiel 11 hergestellt. Man geht aus von 1,4 g (0,0035 mol) Amoxicillintrihydrat sowie dem Umsetzungsprodukt von 650 mg (0,0035 mol) 5-Amino-4-hydroxy-2-(3'-hydroxypropylamino)pyrimidin mit 350 mg Phosgen.

Ausbeute: 1,08 g Natriumsalz (59,5%)

IR-Spektrum: 1770, 1655, 1610, 1540 $cm^{-1}$

NMR-Spektrum (DMSO + $CD_3OD$) Signale bei ppm: 1,55 (d,6H), 1,8 (m,2H), 3,2 bis 3,8 (m,4H), 4,0 (s,1H), 5,4 (q+s,3H), 6,8 (d,2H), 7,3 (d,2H), 8,05 (s,1H).

*Beispiel 14*

*D-α-[3-(4-Hydroxy-2-{2'-hydroxypropyl-amino}-5-pyrimidinyl)ureido-p-hydroxybenzylpenicillinnatrium*

Dieses Penicillin wurde analog Beispiel 11 hergestellt. Man geht aus von 1,68 g (0,004 mol) Amoxicillin trihydrat sowie dem Umsetzungsprodukt von 740 mg (0,004 mol) 5-Amino-4-hydroxy-2-(2'-hydroxypropylamino)pyrimidin mit 400 mg Phosgen (nach Silylierung).

Ausbeute: 920 mg Natriumsalz (38,5%)

IR-Spektrum: 1770, 1660, 1610, 1550 $cm^{-1}$

NMR-Spektrum (DMSO + $CD_3OD$) Signale bei ppm: 1,15 (d,3H), 1,55 (6H), 3,6 (m,2H), 3,9 (m,1H), 4,05 (s,1H), 5,4 (q,2H), 5,5 (s,1H), 6,8 (d,2H), 8,15 (s,1H).

*Beispiel 15*

*D-α-[3-(4-Hydroxy-2-{2'-hydroxypropyl-amino}-5-pyrimidinyl)ureido]-benzylpenicillinnatrium*

Dieses Penicillin wurde analog Beispiel 11 hergestellt. Man geht aus von 800 mg (0,002 mol) Ampicillinnatriumsalz sowie dem Umsetzungsprodukt von 370 mg (0,002 mol) des Pyrimidins des Beispiels 5 mit 200 mg Phosgen.

Ausbeute: 520 mg Natriumsalz (45%)

IR-Spektrum: 1765, 1650, 1610, 1550 $cm^{-1}$

NMR-Spektrum (DMSO + $CD_3OD$) Signale bei ppm: 1,2 (d,3H), 1,55 (d,6H), 3,55 (m,2H), 3,95 (m,1H), 4,05 (s,1H), 5,4 (q,2H), 5,55 (s,1H), 7,4 (5H), 8,15 (s,1H).

*Beispiel 16*

*D-α-[3-(2-{Äthoxycarbonylmethylamino}-4-hydroxy-5-pyrimidinyl)ureido]-p-hydroxybenzylpenicillinnatrium*

Dieses Penicillin wurde analog Beispiel 1 hergestellt. Man geht aus von 1,05 g (0,0025 mol) Amoxicillin trihydrat sowie dem Umsetzungsprodukt von 530 mg (0,0025 mol) 5-Amino-2-äthoxy-carbonylmethylamino-4-hydroxypyrimidin mit 250 mg Phosgen und 0,34 ml Triäthylamin.

Ausbeute: 1,38 g Natriumsalz (88%)

IR-Spektrum: 1770, 1750, 1670, 1620, 1545, 1540 $cm^{-1}$

NMR-Spektrum (DMSO + $CD_3OD$) Signale bei ppm: 1,2 (t,3H), 1,55 (d,6H), 3,5 (q,2H), 4,05 (s,1H), 4,15 (2H), 5,4 (q,2H), 5,45 (s,1H), 6,8 (2H), 7,3 (2H), 8,15 (1H).

*Beispiel 17*

*D-α-[3-(2-{2'-Äthoxycarbonyläthylamino}-4-hydroxy-5-pyrimidinyl)ureido]-p-hydroxybenzylpenicillinnatrium*

Dieses Penicillin wurde analog Beispiel 1 hergestellt. Man geht aus von 840 mg (0,002 mol) Amoxicillintrihydrat sowie dem Umsetzungsprodukt von 500 mg (0,002 mol) 5-Amino-2-(2'-äthoxy-carbonyläthylamino)-4-hydroxypyrimidin mit 200 mg Phosgen und 0,27 ml Triäthylamin.

Ausbeute: 1,02 g Natriumsalz (80%)

IR-Spektrum: 1770, 1750, 1660, 1620, 1545, 1515 $cm^{-1}$

NMR-Spektrum (DMSO + $CD_3OD$) Signale bei ppm: 1,25 (t,3H), 1,55 (d,6H), 3,4 (m,4H), 4,05 (1H), 4,15 (2H), 5,45 (q,2H), 5,50 (s,1H), 6,8 (d,2H), 7,3 (d,2H), 8,15 (s,1H).

*Beispiel 18*

*D-α-[3-(2-{2'-Diäthylaminoäthylamino}-4-hydroxy-5-pyrimidinyl)ureido]-p-hydroxybenzylpenicillinnatrium*

Dieses Penicillin wurde analog Beispiel 1 hergestellt. Man geht aus von 2,1 g (0,005 mol) Amoxicillintrihydrat sowie dem Umsetzungsprodukt von 1,16 g (0,005 mol) 5-Amino-2-(2'-diäthylaminoäthylamino)-4-hydroxypyrimidin mit 500 mg Phosgen und 0,68 ml Triäthylamin. Die Aufarbeitung wurde folgendermaßen abgeändert: Die wäßrige Phase wurde bei pH 3,4 zweimal mit je 50 ml n-Butanol ausgeschüttelt. Das Butanol wurde im Hochvakuum entfernt und der verbleibende Rückstand auf bekannte Weise in das Natriumsalz überführt.

Ausbeute: 1,22 g Natriumsalz (38%)

IR-Spektrum: 1770, 1660, 1610, 1550 $cm^{-1}$

NMR-Spektrum (DMSO + $CD_3OD$) Signale bei ppm: 1,2 bis 1,7 (m,12H), 3,1 bis 3,7 (m,8H), 4,05 (s,1H), 5,5 (m,3H), 6,85 (d,2H), 7,35 (d,2H), 8,20 (s,1H).

*Beispiel 19*

*D-α-[3-(4-Hydroxy-2-{2'-phenoxyäthylamino}-5-pyrimidinyl)ureido]-p-hydroxybenzylpenicillinnatrium*

Dieses Penicillin wurde analog Beispiel 1 hergestellt. Man geht aus von 1,6 g (0,004 mol) Amoxicillintrihydrat sowie dem Umsetzungsprodukt von 985 mg 5-Amino-4-hydroxy-1-(2'-phenoxyäthylamino)pyrimidin mit 500 mg Phosgen und 0,67 ml Triäthylamin.

Ausbeute: 2,46 g Natriumsalz (72,5%)

IR-Spektrum: 1770, 1660, 1610, 1550 $cm^{-1}$

NMR-Spektrum (DMSO + $CD_3OD$) Signale bei ppm: 1,55 (d,6H), 3,7 (m,2H), 4,05 (s,1H), 4,1 (m,2H), 5,45 (m,3H), 6,8 bis 7,4 (m,9H), 8,15 (s,1H).

*Beispiel 20*

*D-α-[3-(2-{2'-Acetylaminoäthylamino}-4-hydroxy-5-pyrimidinyl)ureido]-p-hydroxybenzylpenicillinnatrium*

Dieses Penicillin wurde analog Beispiel 11 hergestellt. Man geht aus von 1,26 g (0,003 mol) Amoxicillintrihydrat sowie dem Umsetzungsprodukt von 630 mg (0,003 mol) 2-(2'-Acetylaminoäthylamino)-5-amino-4-hydroxypyrimidin mit 300 mg Phosgen.

Ausbeute: 865 mg Natriumsalz (46,5%)

IR-Spektrum: 1770, 1660, 1610, 1550 $cm^{-1}$

NMR-Spektrum (DMSO + $CD_3OD$) Signale bei ppm: 1,55 (d,6H), 1,95 (s,3H), 2,7 (m,2H), 3,5 (m,2H), 4,05 (s,1H), 5,45 (m,3H), 6,8 (d,2H), 7,3 (d,2H), 8,05 (s,1H).

*Beispiel 21*

*D-α-[3-(2-{2'-Äthylsulfinyläthylamino}-4-hydroxy-5-pyrimidinyl)ureido]-p-hydroxybenzylpenicillinnatrium*

Dieses Penicillin wurde analog Beispiel 11 hergestellt. Man geht aus von 420 mg (0,001 mol) Amoxicillintrihydrat sowie 230 mg 5-Amino-2-(2'-äthylsulfinyläthylamino)-4-hydroxypyrimidin, das nach Silylierung mit 100 mg Phosgen umgesetzt wurde.

Ausbeute: 265 mg Natriumsalz (41%)

IR-Spektrum: 1765, 1660, 1610, 1545 $cm^{-1}$

NMR-Spektrum (DMSO + $CD_3OD$) Signale bei ppm: 1,3 (t,3H), 1,55 (d,6H), 3,1 (m,4H), 3,5 (m,2H), 4,05 (s,1H), 5,45 (q,2H), 5,50 (s,1H), 6,85 (2H), 7,35 (2H), 8,15 (1H).

*Beispiel 22*

*D-α-[3-(2-{2'-Acetyläthylamino}-4-hydroxy-5-pyrimidinyl)ureido]-p-hydroxybenzylpenicillinnatrium*

Dieses Penicillin wurde analog Beispiel 1 hergestellt. Man geht aus von 4,2 g (0,01 mol) Amoxicillintrihydrat sowie dem Umsetzungsprodukt von 1,96 g (0,01 mol) 2-(2'-Acetyläthylamino)-5-amino-4-hydroxypyrimidin mit 1,01 g Phosgen und 1,35 ml Triäthylamin.

Ausbeute: 3,17 g Natriumsalz (52%)

IR-Spektrum: 1770, 1680, 1660, 1610, 1545 $cm^{-1}$

NMR-Spektrum (DMSO + $CD_3OD$) Signale bei ppm: 1,55 (d,6H), 2,2 (s,3H), 2,7 (m,2H), 3,4 (m,2H), 4,05 (1H), 5,45 (q,2H), 5,50 (s,1H), 6,85 (2H), 7,35 (2H), 8,15 (1H).

*Beispiel 23*

*D-α-[3-(4-Hydroxy-2-{2'-methylmercaptoäthylamino}-5-pyrimidinyl)ureido]-p-hydroxybenzylpenicillinnatrium*

Dieses Penicillin wurde analog Beispiel 1 hergestellt. Man geht aus von 2,1 g (0,005 mol) Amoxicillintrihydrat sowie dem Umsetzungsprodukt von 1,03 g (0,005 mol) 5-Amino-4-hydroxy-2-(2'-methylmercaptoäthylamino)pyrimidin mit 500 mg Phosgen und 0,67 ml Triäthylamin.

Ausbeute: 2,35 g Natriumsalz (75%)

IR-Spektrum: 1770, 1660, 1610, 1550 $cm^{-1}$

NMR-Spektrum (DMSO + $CD_3OD$) Signale bei ppm: 1,55 (d,6H), 2,3 (s,3H), 2,7 (m,2H), 3,4 (m,2H), 4,05 (s,1H), 5,45 (q,2H), 5,5 (s,1H), 6,85 (d,2H), 7,35 (d,2H), 8,15 (s,1H).

*Beispiel 24*

*D-α-[3-(2-{2'-Äthylmercaptoäthylamino}-4-hydroxy-5-pyrimidinyl)ureido]-p-hydroxybenzylpencillinnatrium*

Dieses Penicillin wurde analog Beispiel 1 hergestellt. Man geht aus von 1,26 g (0,003 mol) Amoxicillintrihydrat sowie dem Umsetzungsprodukt von 640 mg (0,003 mol) 5-Amino-2-(2'-äthylmercaptoäthylamino)-4-hydroxypyrimidin mit 300 mg Phosgen und 0,41 ml Triäthylamin. Bei der Aufarbeitung wird das entstehende Penicillin bei pH 2,7 mit Essigsäureäthylester ausgeschüttelt und anschließend wird über Natriumsulfat getrocknet und der Essigsäureäthylester im Vakuum entfernt. Das zurückbleibende Endprodukt wird in üblicher Weise in das Natriumsalz überführt.

Ausbeute: 1,31 g Natriumsalz (69,5%)
IR-Spektrum: 1770, 1660, 1610, 1540, 1515 $cm^{-1}$
NMR-Spektrum (DMSO + $CD_3OD$) Signale bei ppm: 1,25 (t,3H), 1,55 (6H), 2,65 (m,4H), 3,5 (m,2H), 4,05 (s,1H), 5,4 (q,2H), 5,5 (s,1H), 6,8 (2H), 7,3 (2H), 8,15 (1H).

*Beispiel 25*

*D-α-[3-(2-{2'-Cyanopropylamino}-4-hydroxy-5-pyrimidinyl)ureido]-p-hydroxybenzylpenicillinnatrium*

Dieses Penicillin wurde analog Beispiel 11 hergestellt. Man geht aus von 4,2 g (0,01 mol) Amoxicillintrihydrat sowie 1,93 g (0,01 mol) 5-Amino-2-(2'-cyanopropylamino)-4-hydroxypyrimidin, das nach Silylierung mit 1,01 g Phosgen umgesetzt wurde.
Ausbeute: 3,6 g Natriumsalz (59%)
IR-Spektrum: 1765, 1650, 1600, 1545 $cm^{-1}$
NMR-Spektrum (DMSO + $CD_3OD$) Signale bei ppm: 1,2 (d,3H), 1,55 (d,6H), 2,8 (m,1H), 3,5 (m,2H), 5,4 (q,2H), 5,5 (s,1H), 6,8 (d,2H), 7,3 (d,2H), 8,10 (s,1H).

*Beispiel 26*

*D-α-[3-(4-Hydroxy-2-{4'-hydroxycyclohexylamino}-5-pyrimidinyl)ureido]-p-hydroxybenzylpenicillinnatrium*

Dieses Penicillin wurde analog Beispiel 11 hergestellt. Man geht aus von 1,68 g (0,004 mol) Amoxicillintrihydrat sowie dem Umsetzungsprodukt von 500 mg (0,004 mol) 5-Amino-4-hydroxy-2-(4'-hydroxycyclohexylalmino)pyrimidin mit 400 mg Phosgen.
Ausbeute: 1,63 g Natriumsalz (64%)
IR-Spektrum: 1770, 1660, 1615, 1550 $cm^{-1}$
NMR-Spektrum (DMSO + $CD_3OD$) Signale bei ppm: 1,2 bis 2,2 (m,14H), 3,65 (m,1H), 3,95 (m,1H), 4,05 (s,1H), 5,45 (q,2H), 5,50 (s,1H), 6,8 (d,2H), 7,3 (d,2H), 8,15 (s,1H).

*Beispiel 27*

*D-α-[3-(4-Hydroxy-2-{4'-hydroxycyclohexylamino}-5-pyrimidinyl)ureido]benzylpenicillinnatrium*

Dieses Penicillin wurde analog Beispiel 11 hergestellt. Man geht aus von 3,75 g (0,01 mol) Ampicillintrihydrat sowie dem Umsetzungsprodukt von 2,24 g (0,01 mol) des Pyrimidins des Beispiels 26 mit 1,01 g Phosgen.
Ausbeute: 3,62 g Natriumsalz (58%)
IR-Spektrum: 1765, 1655, 1615, 1545 $cm^{-1}$
NMR-Spektrum (DMSO + $CD_3OD$) Signale bei ppm: 1,15 bis 2,1 (m,14H), 3,7 bis 4,0 (m,1H+1H), 4,05 (s,1H), 5,40 (q,2H), 5,55 (s,1H), 7,4 (m,5H), 8,15 (1H).

*Beispiel 28*

*D-α-[3-(2-{2'-Anilinoäthylamino}-4-hydroxy-5-pyrimidinyl)ureido]-p-hydroxybenzylpenicillinnatrium*

Dieses Penicillin wurde analog Beispiel 1 hergestellt. Man geht aus von 2,1 g (0,005 mol) Amoxicillintrihydrat sowie dem Umsetzungsprodukt von 1,23 g (0,005 mol) 5-Amino-2-(2'-anilinoäthylamino)-4-hydroxypyrimidin mit 500 mg Phosgen und 0,68 ml Triäthylamin.
Ausbeute: 2,06 g Natriumsalz (61%)
IR-Spektrum: 1765, 1660, 1610, 1545 $cm^{-1}$
NMR-Spektrum (DMSO + $CD_3OD$) Signale bei ppm: 1,55 (d,6H), 3,2 bis 3,8 (m,4H), 4,05 (1H), 5,45 (q,2H), 5,50 (s,1H), 6,8 bis 7,45 (m,9H), 8,20 (s,1H).

*Beispiel 29*

*D-α-[3-(2-{3'-Aminocarbonylpropylamino}-4-hydroxy-5-pyrimidinyl)ureido]-p-hydroxybenzylpenicillinnatrium*

Herstellung analog Beispiel 11. Man geht aus von 1,1 g (0,0026 mol) Amoxycillintrihydrat sowie 550 mg (0,0025 mol) 5-Amino-2-(3'-aminocarbonylpropylamino)-4-hydroxypyrimidin, das nach Silylierung mit 260 mg Phosgen in Tetrahydrofuran umgesetzt wurde.
Ausbeute: 740 mg Natriumsalz (47%)
IR-Spektrum: 1765, 1660, 1615, 1550 $cm^{-1}$
NMR-Spektrum (DMSO + $CD_3OD$) Signale bei ppm: 1,55 (d,6H), 1,9 (t,2H), 2,8 (m,2H), 3,45 (m,2H), 4,05 (s,1H), 5,45 (q,2H), 5,50 (s,1H), 6,8 (d,2H), 7,35 (d,2H), 8,15 (s,1H).

*Beispiel 30*

*D-α-[3-(2-{3'-Aminosulfonylpropylamino}-4-hydroxy-5-pyrimidinyl) ureido]-p-hydroxybenzylpenicillinnatrium*

Darstellung analog Beispiel 11. 2,1 g (0,005 mol) Amoxycillintrihydrat werden mit dem Reaktionsprodukt von 1,5 g (0,005 mol) 5-Amino-2-(3'-aminosulfonylpropylamino)-4-hydroxypyrimidin, 5 ml Trimethylsilyldiäthylamin und 500 mg Phosgen umgesetzt.
Ausbeute: 1,84 g (56,5%)
IR-Spektrum: 1770, 1660, 1610, 1560, 1300, 1160 $cm^{-1}$
NMR-Spektrum (DMSO + $CD_3OD$) Signale bei ppm: 1,55 (d,6H), 2,0 (m,2H), 2,85 (m,2H), 3,40 (m,2H), 4,05 (s,1H), 5,45 (q,2H), 5,50 (s,1H), 6,85 (d,2H), 7,35 (d,2H), 8,15 (s,1H).

*Beispiel 31*

*D-α-[3-(4-Hydroxy-2-{2'-methoxyäthylamino}-5-pyrimidinyl)ureido]benzylpenicillinnatrium*

Zu einer Suspension von 1,94 g (0,005 mol) D-α-[3-(4-Hydroxy-2-{2'-methoxyäthylamino}-5-pyrimidinyl)ureido]phenylglycinnatriumsalz in 25 ml wasserfreiem Aceton gibt man 10 mg N-Methylmorpholin. Man kühlt auf −20° C bis −15° C ab und tropft bei dieser Temperatur eine Lösung von 550 mg (0,005 mol) Chlorameisensäureäthylester in 10 ml wasserfreien Aceton zu. Man rührt 1 h bei −20° C nach. Anschließend wird bei dieser Temperatur eine Lösung von 1,6 g des Triäthylammoniumsalzes der 6-Aminopenicillansäure in 10 ml wasserfreiem Methylenchlorid zugetropft. Man rührt 1 h bei −20° C, 1 h bei 0° C und 1 h bei Raumtemperatur nach. Das organische Lösungsmittel wird dann im Vakuum abgezogen und der Rückstand in einem Gemisch von 40 ml Wasser und 60 ml Essigsäureäthylester bei pH 7,0 gelöst. Die wäßrige Phase wird abgetrennt und unter Eiskühlung auf pH 2,9 (mit verdünnter Salzsäure) gestellt. Das ausgefallene Festprodukt wird abgesaugt und im Vakuum getrocknet. Mit Natriumäthylhexanoat wird das Natriumsalz hergestellt.

Ausbeute: 2,05 g (70%)

IR-Spektrum: 1770, 1660, 1610, 1545, 1515 $cm^{-1}$

NMR-Spektrum (DMSO + $CD_3OD$) Signale bei ppm: 1,55 (d,6H), 3,3 (s,3H), 3,5 (m,4H), 4,1 (s,1H), 5,5 (q,2H), 5,55 (s,1H), 7,5 (m,5H), 8,1 (s,1H).

*Beispiel 32*

*D-α-[3-(2{2'-Äthylmercaptoäthylamino}-4-hydroxy-5-pyrimidinyl)ureido]benzylpenicillinnatrium*

2,16 g (0,01 mol) wasserfreie 6-Aminopenicillansäure werden mit 2,5 ml N,O-(Bistrimethylsilyl)acetamid bei Raumtemperatur in trockenem Dimethylformamid silyliert. Die entstandene Lösung wird bei −10° C zu einer bei −20° C bereiteten Lösung (siehe auch Beispiel 31) von 3,9 g (0,01 mol) D-α-[3-{2'-Äthylmercaptoäthylamino-4-hydroxy-5-pyrimidinylureido]phenylglycin, 1,1 g Chlorameisensäureäthylester und 1,05 g N-Methylmorpholin getropft. Weitere Umsetzung und Aufarbeitung siehe Beispiel 31.

Ausbeute: 2,8 g Natriumsalz (46%)

IR-Spektrum: 1770, 1660, 1610, 1550 $cm^{-1}$

NMR-Spektrum (DMSO + $CD_3OD$) Signale bei ppm: 1,25 (t,3H), 1,55 (d,6H), 2,7 (m,4H), 3,45 (t,2H), 4,0 (s,1H), 5,4 (q,2H), 5,55 (s,1H), 7,4 (m,5H), 8,15 (s,1H).

*Beispiel 33*

*D,L-α-[3-(2-{3'-Hydroxypropylamino}-4-hydroxy-5-pyrimidinyl)ureido]thienylmethylpenicillinnatrium*

Herstellung analog Beispiel 32, ausgehend von 1,84 g (0,005 mol) der Ureidocarbonsäure des Beispiels D sowie 1,08 g (0,005 mol) 6-Aminopenicillansäure.

Ausbeute: 1,53 g (52,5%)

IR-Spektrum: 1765, 1660, 1615, 1550 $cm^{-1}$

NMR-Spektrum (DMSO + $CD_3OD$) Signale bei ppm: 1,55 (d,6H), 1,8 (m,2H), 3,5 (m,4H), 4,05 (s,1H), 5,5 (m,2H), 5,7 (s,1H), 6,9 (breites s,2H), 7,30 (d,1H), 8,1 (s,1H).

**III. Darstellung pharmazeutischer Anwendungsformen:**

*Beispiel I*

*Tabletten enthaltend D-α-[3-(4-Hydroxy-2-{3'-hydroxypropylamino}-5-pyrimidinyl)-ureido]-p-hydroxybenzylpenicillinnatrium*

Ein Gemisch bestehend aus 2 kg Wirksubstanz, 5 kg Lactose, 1,8 kg Kartoffelstärke, 0,1 kg Magnesiumstearat und 0,1 kg Talk wird in üblicher Weise zu Tabletten gepreßt, derart, daß jede Tablette 200 mg Wirkstoff enthält.

*Beispiel II*

*Dragées enthaltend D-α-[3-(4-Hydroxy-2-{3'-hydroxypropylamino}-5-pyrimidinyl)-ureido]-p-hydroxybenzylpenicillinnatrium*

Analog Beispiel I werden Tabletten gepreßt, die anschließend in üblicher Weise mit einem Überzug, bestehend aus Zucker, Kartoffelstärke, Talk und Tragant überzogen werden.

*Beispiel III*

*Kapseln enthaltend D-α-[3-(4-Hydroxy-2-{3'-hydroxypropylamino}-5-pyrimidinyl)-ureido]-p-hydroxybenzylpenicillinnatrium*

5 kg Wirksubstanz werden in üblicher Weise in Hartgelatinekapseln gefüllt, derart, daß jede Kapsel 500 mg des Wirkstoffes enthält.

*Beispiel IV*

*Trockenampullen enthaltend D-α-[3-(4-Hydroxy-2-{3'-hydroxypropylamino}-5-pyrimidinyl)ureido]-p-hydroxybenzylpenicillinnatrium*

In einem aseptischen Bereich wurde 251 g Wirkstoff in 2008 ml destilliertem Wasser zur Injektion aufgelöst. Die Lösung wurde durch ein Millipore-Filter (Porengröße 0,22 μm, Produkt der Millipore Corporation, Bedford, USA) filtriert. Die Lösung wurde jeweils in einer Menge von 2,0 ml in 1000 Gläschen (Kapazität 10 ml) eingegossen und sie wurde liophilisiert. Die Gläschen wurden sodann mit einem Kautschukstöpsel und einer Aluminiumkappe verschlossen. Somit wurden Gläschen (Nr. A) jeweils mit 250 mg Wirkstoff erhalten.

Eine physiologische Kochsalzlösung zur Injektion wurde in einer Menge von jeweils 2,0 ml in Ampullen abgefüllt, und die Ampullen wurden verschlossen. Auf diese Weise wurden Ampullen (Nr. B) erhalten. Die physiologische Kochsalzlösung in den Ampullen (Nr. B) wurde in die Gläschen (Nr. A) gegossen, wodurch eine injizierbare

Zubereitung für die intravenöse Verabreichung erhalten wurde.

Destilliertes Wasser zur Injektion wurde in einer Menge von 20 ml in die Gläsche (Nr. A) gegossen, und die Lösung wurde in einer 5%igen Lösung von Glucose für Injektionen (250 ml) aufgelöst. Auf diese Weise wurden Lösungen für die kontinuierliche Infusion hergestellt.

Analog sind Tabletten, Dragées, Kapseln und Ampullen erhältlich, die einen oder mehrere der übrigen Wirkstoffe der Formel I oder die physiologisch unbedenklichen Salze dieser Verbindungen enthalten.

**Patentansprüche für die Vertragsstaaten:** BE, CH, DE, FR, GB, IT, LU, NL, SE

1. Neue Penicilline der allgemeinen Formel I,

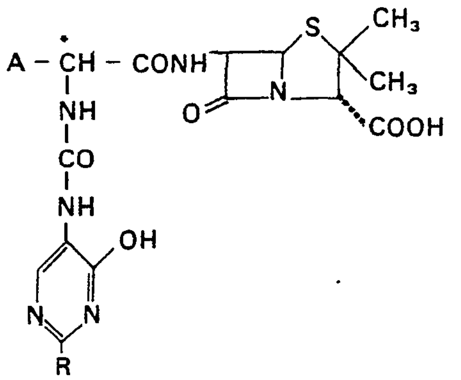

(I)

bzw. der Tautomeren der allgemeinen Formel I',

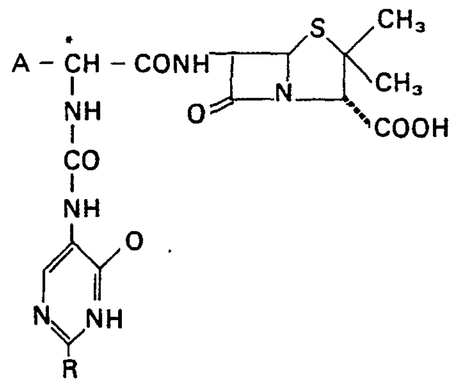

(I')

in denen

A die Phenyl-, 4-Hydroxyphenyl- oder die 2- oder 3-Thienylgruppe sowie einen in 3,4-Stellung disubstituierten Phenylrest, wobei die Substituenten gleich oder verschieden und Chloratome, Hydroxy- oder Methoxygruppen sein können, und

R eine Gruppe der allgemeinen Formel:

$NH-Z-X$ bedeuten,

in der

Z eine geradkettige oder verzweigte Alkylengruppe mit 1 bis 6 Kohlenstoffatomen oder eine durch den Substituenten X substituierte Cycloalkylgruppe mit 3 bis 6 Kohlenstoffatomen und

X die Cyanogruppe, die Hydroxygruppe, die Mercaptogruppe, die Aminocarbonyl- oder Aminosulfonylgruppe oder eine Gruppe der allgemeinen Formeln:

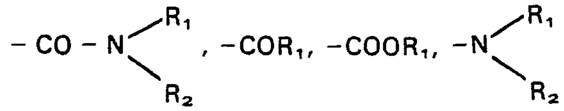

, $-COR_1$, $-COOR_1$, ,

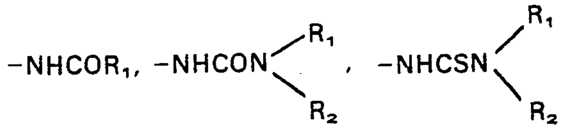

$-NHCOR_1$, , ,

$-NHSO_2R_1$, $-OR_1$, $-OCOR_1$, $-SR_1$, $-SOR_1$ und $-SO_2R_1$

darstellen, wobei in diesen Formeln $R_1$ eine geradkettige oder verzweigte Alkylgruppe mit 1 bis 4 Kohlenstoffatomen oder die Phenylgruppe und $R_2$ ein Wasserstoffatom oder eine Alkylgruppe mit 1 bis 4 Kohlenstoffatomen darstellen, oder $R_1$ und $R_2$ auch zusammen mit einem eventuell dazwischenliegenden Stickstoffatom einen 3- bis 6gliedrigen monocyclischen, heterocyclischen Ring bilden können, und deren physiologisch verträglichen Salze mit anorganischen oder organischen Basen.

2. Neue Penicilline der allgemeinen Formel I:

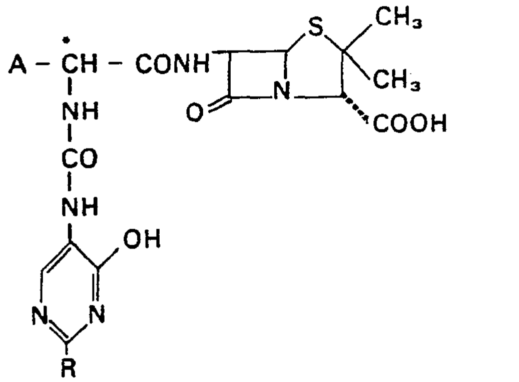

(I)

bzw. der Tautomeren der allgemeinen Formel I':

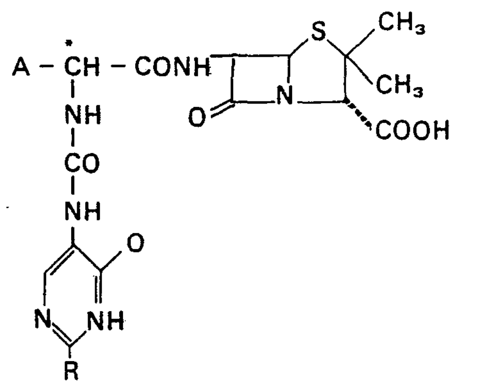

(I')

worin

A die Phenyl- oder p-Hydroxyphenylgruppe und R eine Gruppe der Formel $-NH-Z-X$ bedeuten, wobei in dieser Formel $-NH-Z-$ eine Gruppe der Formeln $-NH-CH_2-CH_2-$, $-NH(CH_2)_3-$,

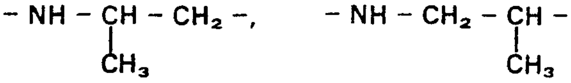

oder 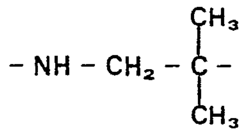 darstellt

und

X die Hydroxy-, Methoxy-, Äthoxy-, Methoxycarbonyl-, Äthoxycarbonyl-, Methylcarbonyl-, Äthylcarbonyl-, Methylmercapto-, Äthylmercapto-, Aminosulfonyl-, Aminocarbonyl-, Acetylamino-, Methylsulfinyl- oder Äthylsulfinylgruppe bedeutet oder R den 4'-Hydroxycyclohexylaminorest darstellt.

3. Neue Penicillin der allgemeinen Formel I bzw. der tautomeren Formel I' gemäß den Ansprüchen 1 und 2, für welche die D=R-Konfiguration zutrifft, und deren physiologisch verträgliche Salze mit anorganischen oder organischen Basen.

4. Verfahren zur Herstellung neuer Penicilline der allgemeinen Formel I:

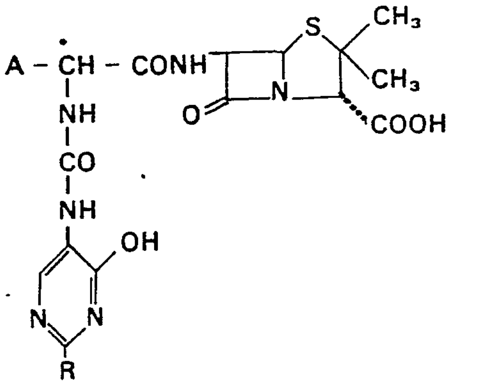 (I)

bzw. der Tautomeren der allgemeinen Formel I':

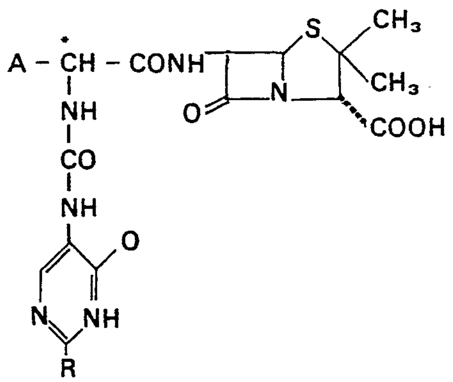 (I')

in denen

A die Phenyl-, 4-Hydroxyphenyl- oder die 2- oder 3-Thienylgruppe sowie einen in 3,4-Stellung disubstituierten Phenylrest, wobei die Substituenten gleich oder verschieden und Chloratome, Hydroxy- oder Methoxygruppen sein können, und

R eine Gruppe der allgemeinen Formel

NH–Z–X bedeuten,

in der

Z eine geradkettige oder verzweigte Alkylengruppe mit 1 bis 6 Kohlenstoffatomen oder eine durch den Substituenten X substituierte Cycloalkylgruppe mit 3 bis 6 Kohlenstoffatomen, und

X die Cyanogruppe, die Hydroxygruppe, die Mercaptogruppe, die Aminocarbonyl- oder Aminosulfonylgruppe oder eine Grupp der allgemeinen Formeln:

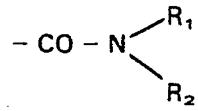, $-COR_1$, $-COOR_1$, 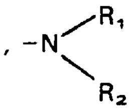,

$-NHCOR_1$, 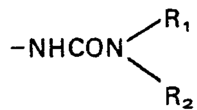, 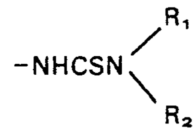,

$-NHSO_2R_1$, $-OR_1$, $-OCOR_1$, $-SR_1$, $-SOR_1$ und $-SO_2R_1$ darstellen, wobei in diesen Formeln $R_1$ eine geradkettige oder verzweigte Alkylgruppe mit 1 bis 4 Kohlenstoffatomen oder die Phenylgruppe und $R_2$ ein Wasserstoffatom oder eine Alkylgruppe mit 1 bis 4 Kohlenstoffatomen darstellen, oder $R_1$ und $R_2$ auch zusammen mit einem eventuell dazwischenliegenden Stickstoffatom einen 3- bis 6-gliedrigen monocyclischen, heterocyclischen Ring bilden können, und von deren physiologisch verträglichen Salzen mit anorganischen oder organischen Basen, dadurch gekennzeichnet, daß

a) eine Verbindung der allgemeinen Formel III:

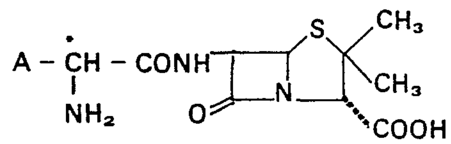 (III)

in der

A wie oben definiert ist, mit einem Pyrimidinderivat der allgemeinen Formel IV:

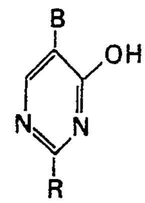 (IV)

in der

R wie oben definiert ist und B die Gruppe $-NCO$ oder ein reaktives Derivat der Gruppe $-NHCOOH$ bedeutet, in einem Lösungsmittel bei Temperaturen zwischen –20 und +50°C umgesetzt wird, oder

b) eine Ureidocarbonsäure der allgemeinen Formel V:

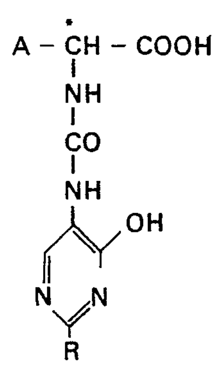 (V)

in der

A und R die oben angegebenen Bedeutungen

besitzen, bzw. ihre Salze oder reaktiven Derivate mit der 6-Aminopenicillansäure der Formel VI:

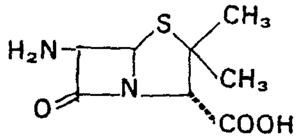

(VI)

oder ihren anorganischen oder organischen Salzen oder reaktiven Derivaten zur Reaktion gebracht werden und, gegebenenfalls anschließend, das so erhaltene Produkt zu einem Penicillin der allgemeinen Formel I bzw. I' hydrolisiert oder katalytisch hydrogenolysiert wird und/oder gewünschtenfalls anschließend das erhaltene Penicillin der allgemeinen Formel I bzw. I' mittels anorganischer oder organischer Basen in sein Salz übergeführt wird.

5. Verfahren gemäß Anspruch 4a, dadurch gekennzeichnet, daß ein Pyrimidinderivat der allgemeinen Formel IV gemäß Anspruch 4a, in der B die Gruppe –NCO oder in der B ein reaktives Derivat der Gruppe –NHCOOH bedeutet, oder, im letzteren Fall, ein Gemisch eines solchen Pyrimidinderivates mit einem anderen Pyrimidinderivat der allgemeinen Formel IV, in der B die Gruppe –NCO darstellt, verwendet wird, und die Umsetzung entweder, bei Vorliegen eines anorganischen oder organischen Salzes einer Verbindung der allgemeinen Formel III, in Mischungen vom Wasser mit in Wasser mischbaren organischen Lösungsmitteln bzw. in organischen Lösungsmitteln bzw. in Gemengen aus Wasser und mit Wasser nichtmischbaren organischen Lösungsmitteln in einem pH-Bereich zwischen 2,0 und 9,0, bzw. in Wasser als Lösungsmittel in Gegenwart einer Base bzw. einer Pufferlösung oder, bei Vorliegen eines Silylderivates oder Carboxylderivates, einer Verbindung der allgemeinen Formel III, in wasser- und hydroxylgruppenfreien Lösungsmitteln, gegebenfalls unter Zusatz von Basen, durchgeführt wird.

6. Verfahren gemäß Anspruch 4b, dadurch gekennzeichnet, daß als reaktive Derivate der Ureidocarbonsäuren der allgemeinen Formel V deren Säureanhydride, deren reaktive Ester oder Amide, deren Säurehalogenide oder Säureazide verwendet werden, und die 6-Aminopenicillansäure der Formel VI in Form ihrer Silylester, Tritylester, p-Nitrobenzylester, Phenacylester und O,N-bis-Trimethylsilylderivate in einem aprotischen Lösungsmittel, in Form ihrer Salze jedoch in einem protischen Lösungsmittel, in Methylenchlorid oder in einem wässerigen Medium oder in einem wässerig-organischen Lösungsmittel bei Temperaturen zwischen –40 und +40°C, gegebenenfalls in Gegenwart einer Base und/oder eines Kondensationsmittels, zur Reaktion gebracht wird und, falls das Endprodukt noch als Ester vorliegt, die Estergruppe anschließend hydrolysiert oder hydrogenolysiert wird.

7. Arzneimittel enthaltend eine oder mehrere Verbindungen gemäß einem der beiden Ansprüche 1 oder 2 neben den üblichen Träger- und/oder Hilfsstoffen.

8. Arzneimittel enthaltend zumindest eine Verbindung gemäß einem der beiden Ansprüche 1 oder 2 neben einem Aminoglykosidantibiotikum oder dessen pharmakologisch verträglichem Salz und einem üblichen Träger- und/oder Hilfsstoff.

**Patentansprüche für den Vertragsstaat:** AT

1. Verfahren zur Herstellung neuer Penicilline der allgemeinen Formel I:

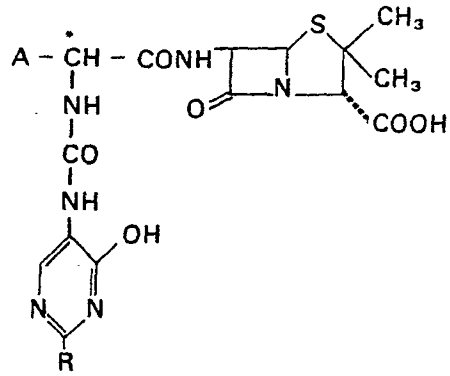

(I)

bzw. der Tautomeren der allgemeinen Formel I':

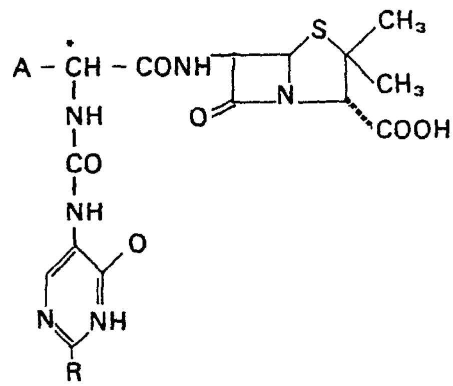

(I')

in denen A die Phenyl-, 4-Hydroxyphenyl- oder die 2- oder 3-Thienylgruppe sowie einen in 3,4-Stellung disubstituierten Phenylrest, wobei die Substituenten gleich oder verschieden und Chloratome, Hydroxy-, oder Methoxygruppen sein können, und

R eine Gruppe der allgemeinen Formel

NH–Z–X bedeuten,

in der

Z eine geradkettige oder verzweigte Alkylengruppe mit 1 bis 6 Kohlenstoffatomen oder eine durch den Substituenten X substituierte Cycloalkylgruppe mit 3 bis 6 Kohlenstoffatomen, und

X die Cyanogruppe, die Hydroxygruppe, die Mercaptogruppe, die Aminocarbonyl- oder Aminosulfonylgruppe oder eine Gruppe der allgemeinen Formeln:

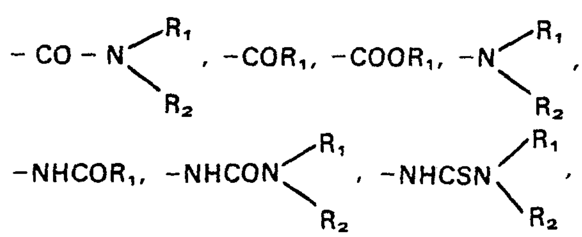

$-NHSO_2R_1$, $-OR_1$, $-OCOR_1$, $-SR_1$, $-SOR_1$ und $-SO_2R_1$ darstellen, wobei in diesen Formeln $R_1$ eine geradkettige oder verzweigte Alkylgruppe mit 1 bis 4 Kohlenstoffatomen oder die Phenylgruppe und $R_2$ ein Wasserstoffatom oder eine Alkylgruppe mit 1 bis 4 Kohlenstoffatomen darstellen, oder $R_1$ und $R_2$ auch zusammen mit einem eventuell dazwischenliegenden Stickstoffatom einen 3- bis 6gliedrigen monocyclischen, heterocyclischen Ring bilden können, und von deren physiologisch verträglichen Salzen mit anorganischen oder organischen Basen, dadurch gekennzeichnet, daß

a) eine Verbindung der allgemeinen Formel III:

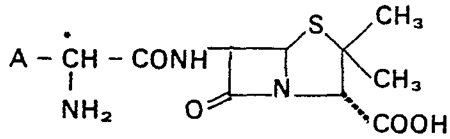

(III)

in der A wie oben definiert ist, mit einem Pyrimidinderivat der allgemeinen Formel IV:

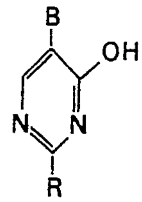

(IV)

in der R wie oben definiert ist und B die Gruppe $-NCO$ oder ein reaktives Derivat der Gruppe $-NHCOOH$ bedeutet, in einem Lösungsmittel bei Temperaturen zwischen $-20$ und $+50°C$ umgesetzt wird, oder

b) eine Ureidocarbonsäure der allgemeinen Formel V:

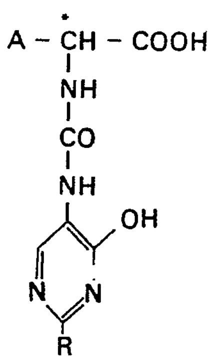

(V)

in der

A und R die oben angegebenen Bedeutungen besitzen, bzw. ihre Salze oder reaktiven Derivate mit der 6-Aminopenicillansäure der Formel VI:

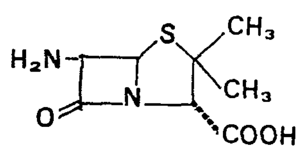

(VI)

oder ihren anorganischen oder organischen Salzen oder reaktiven Derivaten zur Reaktion gebracht werden und, gegebenenfalls anschließend, das so erhaltene Produkt zu einem Penicillin der allgemeinen Formel I bzw. I' hydrolisiert oder katalytisch hydrogenolysiert wird und/oder gewünschtenfalls anschließend das erhaltene Penicillin der allgemeinen Formel I bzw. I' mittels anorganischer oder organischer Basen in sein Salz übergeführt wird.

2) Verfahren zur Herstellung neuer Penicilline der allgemeinen Formel I:

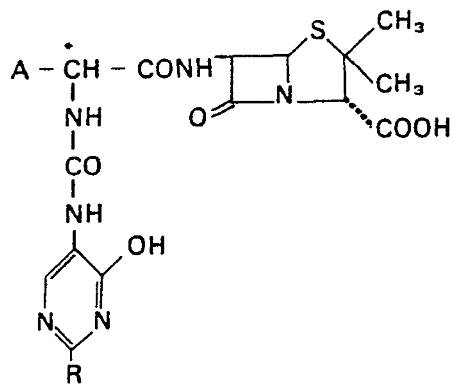

(I)

bzw. der Tautomeren der allgemeinen Formel I':

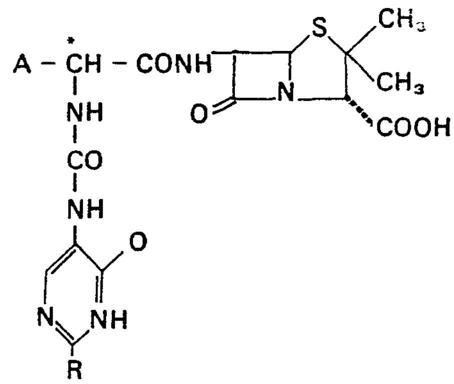

(I')

in denen A die Phenyl-, 4-Hydroxyphenyl- oder die 2- oder 3-Thienylgruppe sowie einen in 3,4-Stellung disubstituierten Phenylrest, wobei die Substituenten gleich oder verschieden und Chloratome, Hydroxy- oder Methoxygruppen sein können, und

R eine Gruppe der allgemeinen Formel

NH–Z–X bedeuten,

in der

Z eine geradkettige oder verzweigte Alkylengruppe mit 1 bis 6 Kohlenstoffatomen oder eine durch den Substituenten X substituierte Cycloalkylgruppe mit 3 bis 6 Kohlenstoffatomen, und

X die Cyanogruppe, die Hydroxygruppe, die Mercaptogruppe oder eine Gruppe der allgemeinen Formeln

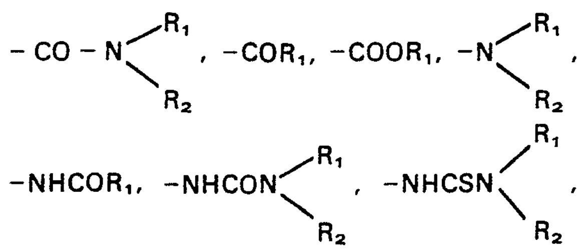

$-NHSO_2R_1$, $-OR_1$, $-OCOR_1$, $-SR_1$, $-SOR_1$ und $-SO_2R_1$ darstellen, wobei in diesen Formeln $R_1$ eine geradkettige oder verzweigte Alkylgruppe mit 1 bis 4 Kohlenstoffatomen oder die

Phenylgruppe und $R_2$ ein Wasserstoffatom oder eine Alkylgruppe mit 1 bis 4 Kohlenstoffatomen darstellen, oder $R_1$ und $R_2$ auch zusammen mit einem eventuell dazwischenliegenden Stickstoffatom einen 3- bis 6gliedrigen monocyclischen, heterocyclischen Ring bilden können, und von deren physiologisch verträglichen Salzen mit anorganischen oder organischen Basen, dadurch gekennzeichnet, daß

a) eine Verbindung der allgemeinen Formel III:

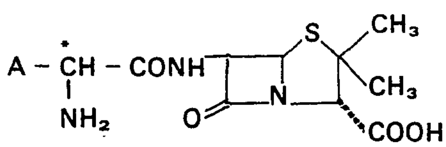

(III)

in der A wie oben definiert ist, mit einem Pyrimidinderivat der allgemeinen Formel IV:

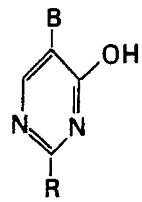

(IV)

in der R wie oben definiert ist und B die Gruppe –NCO oder ein reaktives Derivat der Gruppe –NHCOOH bedeutet, in einem Lösungsmittel bei Temperaturen zwischen –20 und +50°C umgesetzt wird, oder

b) eine Ureidocarbonsäure der allgemeinen Formel V:

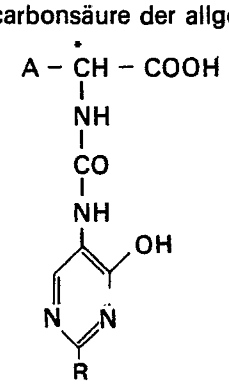

(V)

in der

A und R die oben angegebenen Bedeutungen besitzen, bzw. ihre Salze oder reaktiven Derivate mit der 6-Aminopenicillansäure der Formel VI:

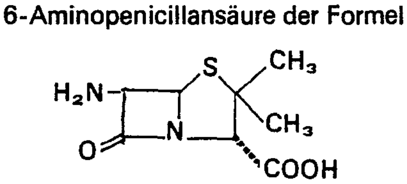

(VI)

oder ihren anorganischen oder organischen Salzen oder reaktiven Derivaten zur Reaktion gebracht werden und, gegebenenfalls anschließend, das so erhaltene Produkt zu einer Penicillin der allgemeinen Formel I bzw. I′ hydrolysiert oder katalytisch hydrogenolysiert wird und/oder gewünschtenfalls anschließend das erhaltene Penicillin der allgemeinen Formel I bzw. I′ mittels anorganischer oder organischer Basen in sein Salz übergeführt wird. (Priorität der deutschen Patentanmeldung P 29 10 190.4 vom 15.3.1979.)

3) Verfahren gemäß der beiden Ansprüchen 1a und 2a, dadurch gekennzeichnet, daß ein Pyrimidinderivat der allgemeinen Formel IV gemäß Anspruch 1a und 2a, in der B die Gruppe –NCO oder in der B ein reaktives Derivat der Gruppe –NHCOOH bedeutet, oder, im letzteren Fall, ein Gemisch eines solchen Pyrimidinderivates mit einem anderen Pyrimidinderivat der allgemeinen Formel IV, in der B die Gruppe –NCO darstellt, verwendet wird und die Umsetzung entweder, bei Vorliegen eines anorganischen oder organischen Salzes einer Verbindung der allgemeinen Formel III, in Mischungen vom Wasser mit in Wasser mischbaren organischen Lösungsmitteln bzw. in organischen Lösungsmitteln bzw. in Gemengen aus Wasser und mit Wasser nichtmischbaren organischen Lösungsmitteln in einem pH-Bereich zwischen 2,0 und 9,0, bzw. in Wasser als Lösungsmittel in Gegenwart einer Base bzw. einer Pufferlösung oder, bei Vorliegen eines Silylderivates oder Carboxylderivates, einer Verbindung der allgemeinen Formel III, in wasser- und hydroxylgruppenfreien Lösungsmitteln, gegebenenfalls unter Zusatz von Basen, durchgeführt wird.

4) Verfahren gemäß der beiden Ansprüchen 1b und 2b, dadurch gekennzeichnet, daß als reaktive Derivate der Ureidocarbonsäuren der allgemeinen Formel V deren Säureanhydride, deren reaktive Ester oder Amide, deren Säurehalogenide oder Säureazide verwendet werden, und die 6-Aminopen cillansäure der Formel VI in Form ihrer Silylester, Tritylester, p-Nitrobenzylester, Phenacylester und O,N-bis-Trimethylsilylderivate in einem aprotischen Lösungsmittel, in Form ihrer Salze jedoch in einem protischen Lösungsmittel, in Methylenchlorid oder in einem wässerigen Medium oder in einem wässerig-organischen Lösungsmittel bei Temperaturen zwischen –40 und +40°C, gegebenenfalls in Gegenwart einer Base und/oder eines Kondensationsmittels, zur Reaktion gebracht wird und, falls das Endprodukt noch als Ester vorliegt, die Estergruppe anschließend hydrolysiert oder hydrogenolysiert wird.

**Claims for the contracting states:** BE, CH, DE, FR, GB, IT, LU, NL, SE

1. New penicillins of general formula I:

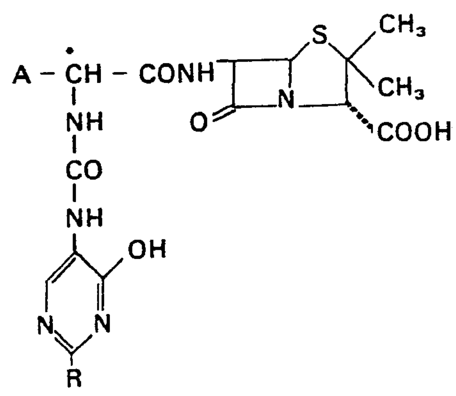

(I)

or the tautomers of general formula I′:

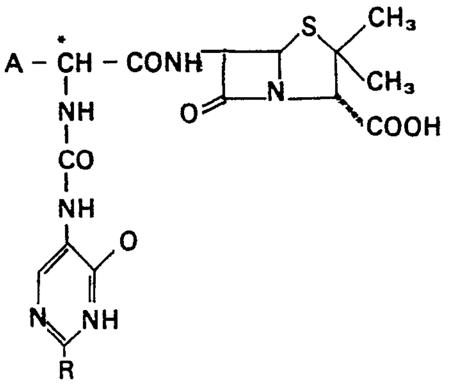

(I')

wherein:

A represents the phenyl, 4-hydroxyphenyl or 2- or 3-thienyl group, or a phenyl radical disubstituted in the 3,4-positions, where the substituents may be the same or different and may represent chlorine atoms, hydroxy or methoxy groups, and

R represents a group of general formula:

NH–Z–X

wherein:

Z represents a straight-chained or branched alkylene group with 1 to 6 carbon atoms or a cycloalkyl group with 3 to 6 carbon atoms substituted by the substituent X, and

X represents the cyano group, the hydroxy group, the mercapto group, the aminocarbonyl or aminosulfonyl group or a group of general formula:

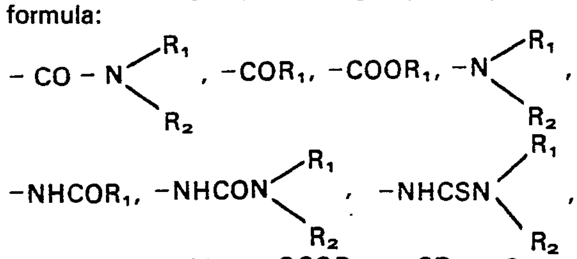

$-NHSO_2R_1$, $-OR_1$, $-OCOR_1$, $-SR_1$, $-SOR_1$ or $-SO_2R_1$,

and, in these formulae, $R_1$ represents a straight-chained or branched alkyl group with 1 to 4 carbon atoms or the phenyl group,

$R_2$ represents a hydrogen atom or an alkyl group with 1 to 4 carbon atoms, and

$R_1$ and $R_2$ may also, together with any nitrogen atom located between them, form a 3- to 6-membered monocyclic, heterocyclic ring, and the physiologically acceptable salts thereof with inorganic or organic bases.

2. New penicillins of general formula I:

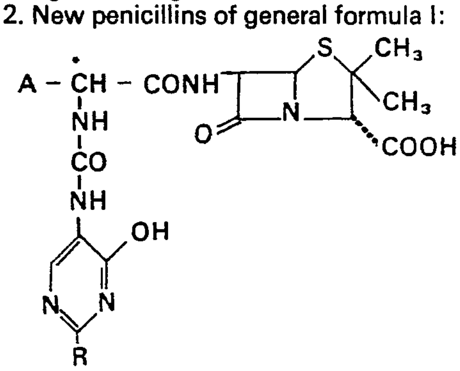

(I)

or the tautomers of general formula I':

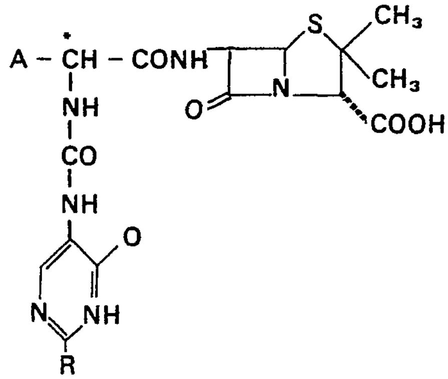

(I')

wherein:

A represents the phenyl or p-hydroxyphenyl group, and

R represents a group of formula –NH–Z–X, in which formula –NH–Z– represents a group of formula:

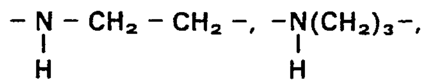

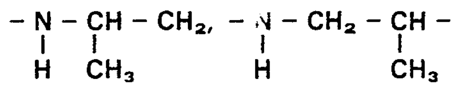

or 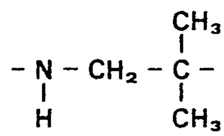 and

X represents the hydroxy, methoxy, ethoxy, methoxycarbonyl, ethoxycarbonyl, methylcarbonyl, ethylcarbonyl, methylmercapto, ethylmercapto, aminosulfonyl, aminocarbonyl, acetylamino, methylsulfinyl or ethylsulfinyl group, or

R represents the 4'- hydroxycyclohexylamino radical.

3. New penicillins of general formula I or of the tautomeric formula I' as claimed in claims 1 and 2, which are present in the D=R configuration, and the physiologically acceptable salts thereof with inorganic or organic bases.

4. Process for the preparation of new penicillins of general formula I:

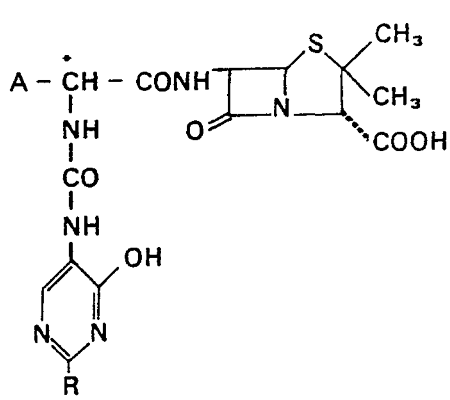

(I)

or the tautomers of general formula I':

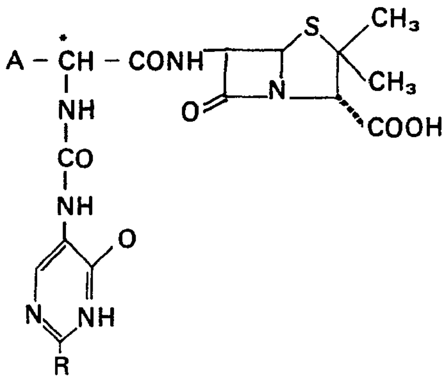

(I')

wherein:

A represents the phenyl, 4-hydroxyphenyl or 2- or 3-thienyl group or a phenyl radical disubstituted in the 3,4-position, where the substituents may be the same or different and may represent chlorine atoms, hydroxy or methoxy groups, and

R represents a group of general formula:

NH–Z–X

wherein:

Z represents a straight-chained or branched alkylene group with 1 to 6 carbon atoms or a cycloalkyl group with 3 to 6 carbon atoms substituted by the substituent X, and

X represents the cyano group, the hydroxy group, the mercapto group, the aminocarbonyl or aminosulfonyl group or a group of general formula:

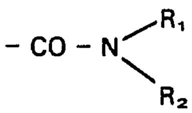, $-COR_1$, $-COOR_1$,

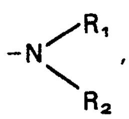, $-NHCOR_1$, 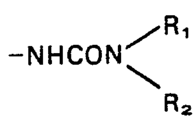,

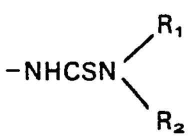, $-NHSO_2R_1$, $-OR_1$,

$-OCOR_1$, $-SR_1$, $-SOR_1$ and $-SO_2R_1$

and, in these formulae, $R_1$ represents a straight-chained or branched alkyl group with 1 to 4 carbon atoms or the phenyl group, and

$R_2$ represents a hydrogen atom or an alkyl group with 1 to 4 carbon atoms, and

$R_1$ and $R_2$ may also, together with any nitrogen atom located between them, form a 3- to 6-membered monocyclic, heterocyclic ring, and the physiologically acceptable salts thereof with inorganic or organic bases, characterised in that:

a) a compound of general formula III:

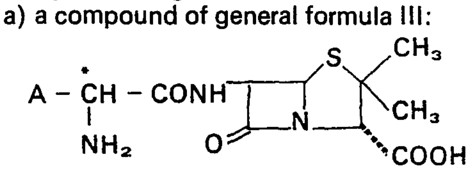

(III)

wherein A is as hereinbefore defined, is reacted with a pyrimidine derivative of general formula IV:

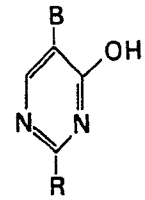

(IV)

wherein

R is as hereinbefore defined, and

B represents the group –NCO or a reactive derivative of the group –NHCOOH, in a solvent at temperatures between –20 and +50° C, or

b) a ureidocarboxylic acid of general formula V:

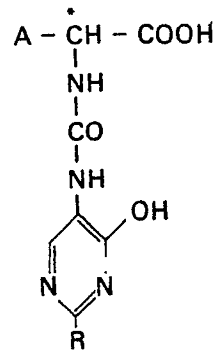

(V)

wherein A and R are as hereinbefore defined, or a salt or reactive derivative thereof, is reacted with 6-aminopenicillanic acid of formula VI:

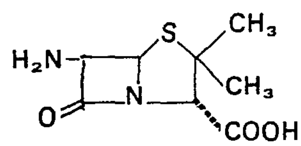

(VI)

or the inorganic or organic salts or reactive derivatives thereof and, if desired, the product thus obtained is subsequently hydrolysed or catalytically hydrogenated to form a penicillin of general formula I or I' and/or, if desired, the penicillin of general formula I or I' thus obtained is subsequently converted into a salt by means of inorganic or organic bases.

5) A process as claimed in claim 4a, characterised in that a pyrimidine derivative of general formula IV as claimed in claim 4a is used, wherein B represents the group –NCO or wherein B represents a reactive derivative of the group –NHCOOH or, in the latter case, a mixture of such a pyrimidine derivative with another pyrimidine derivative of general formula IV wherein B represents the group –NCO is used, and when an inorganic or organic salt of a compound of general formula III is present, the reaction is effected in mixtures of water and water-miscible organic solvents, or in organic solvents, or in mixtures of water and water-immiscible organic solvents in a pH range of between 2.0 and 9.0, or in water as the solvent, in the presence of a base or a buffer solution, or, when a silyl derivative or carboxyl derivative of a compound of general formula III is present, the reaction is effected in anhydrous solvents or solvents free from hydroxyl groups, optionally with the addition of bases.

6) A process as claimed in claim 4b, characterised

in that the reactive derivatives of the ureidocarboxylic acids of general formula V used are the acid anhydrides, the reactive esters or amides, the acid halides or acid azides thereof, and the 6-aminopenicillanic acid of formula VI is reacted in the form of its silyl esters, trityl esters, p-nitrobenzyl esters, phenacyl esters and O,N-bis-trimethylsilyl derivatives in an aprotic solvent, but, when used in the form of its salts, it is reacted in a protic solvent, in methylene chloride or in an aqueous medium or in an aqueous organic solvent at temperatures of between −40 and +40°C, optionally in the presence of a base and/or a condensing agent, and, if the end product is still present as an ester, the ester group is subsequently split off by hydrolysis or hydrogenolysis.

7) Pharmaceutical compositions containing one or more compounds as claimed in claim 1 or 2, together with the usual carriers and/or excipients.

8) Pharmaceutical compositions containing at least one compound as claimed in claim 1 or 2, together with an aminoglycoside antibiotic or the pharmacologically acceptable salt thereof and a conventional carrier and/or excipient.

**Claims for the contracting state:** AT

1. Process for the preparation of new penicillins of general formula I:

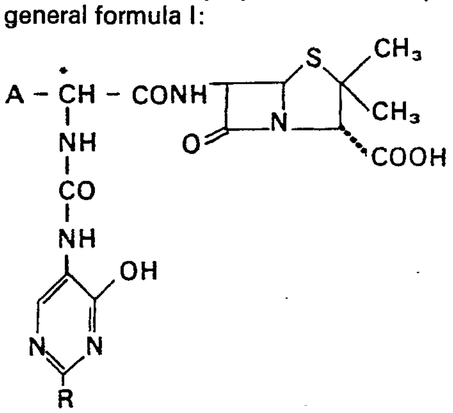

(I)

or the tautomers of general formula I':

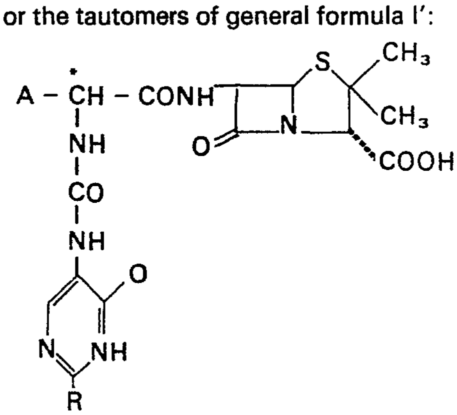

(I')

wherein:

A represents the phenyl, 4-hydroxyphenyl or 2- or 3-thienyl group or a phenyl radical disubstituted in the 3,4-position, where the substituents may be the same or different and may represent chloride atoms, hydroxy or methoxy groups, and

R represents a group of general formula:

NH–Z–X

wherein:

Z represents a straight-chained or branched alkylene group with 1 to 6 carbon atoms or a cycloalkyl group with 3 to 6 carbon atoms substituted by the substituent X, and

X represents the cyano group, the hydroxy group, the mercapto group, the aminocarbonyl or aminosulfonyl group or a group of general formula:

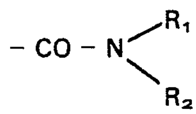 , $-COR_1$, $-COOR_1$,

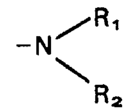 , $-NHCOR_1$, 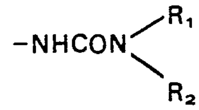 ,

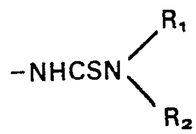 ,

$-NHSO_2R_1$, $-OR_1$, $-OCOR_1$, $-SR_1$, $SOR_1$ or $-SO_2R_1$

and in these formulae, $R_1$ represents a straight-chained or branched alkyl group with 1 to 4 carbon atoms or the phenyl group,

$R_2$ represents a hydrogen atom or an alkyl group with 1 to 4 carbon atoms, and

$R_1$ and $R_2$ may also, together with any nitrogen atom located between them, form a 3- to 6-membered monocyclic, heterocyclic ring, and the physiologically acceptable salts thereof with inorganic or organic bases, characterised in that:

a) a compound of general formula III:

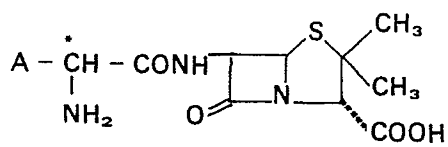

(III)

wherein A is as hereinbefore defined, is reacted with a pyrimidine derivative of general formula IV:

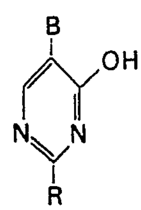

(IV)

wherein

R is as hereinbefore defined, and

B represents the group −NCO or a reactive derivative of the group −NHCOOH, in a solvent at temperatures between −20 and +50°C, or

b) a ureidocarboxylic acid of general formula V:

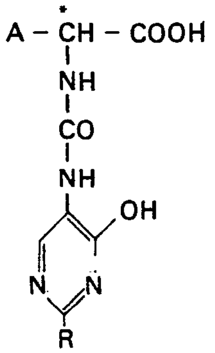

(V)

wherein A and R are as hereinbefore defined, or a salt or reactive derivative thereof, is reacted with 6-aminopenicillanic acid of formula VI:

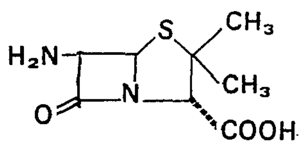

(VI)

or the inorganic or organic salts or reactive derivatives thereof and, if desired, the product thus obtained is subsequently hydrolysed or catalytically hydrogenated to form a penicillin of general formula I or I' and/or, if desired, the penicillin of general formula I or I' thus obtained is subsequently converted into a salt by means of inorganic or organic bases.

2. Process for the preparation of new penicillins of general formula I:

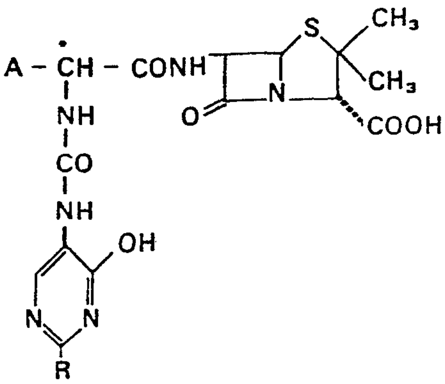

(I)

or the tautomers of general formula I':

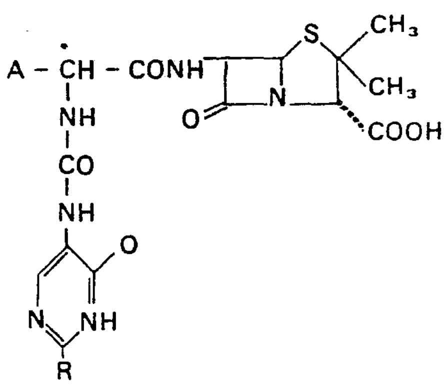

(I')

wherein:

A represents the phenyl, 4-hydroxyphenyl or 2- or 3-thienyl group or a phenyl radical disubstituted in the 3,4-position, where the substituents may be the same or different and may represent chlorine atoms, hydroxy or methoxy groups, and

R represents a group of general formula:

NH–Z–X

wherein:

Z represents a straight-chained or branched alkylene group with 1 to 6 carbon atoms or a cycloalkyl group with 3 to 6 carbon atoms substituted by the substituent X, and

X represents the cyano group, the hydroxy group, the mercapto group, or a group of general formula:

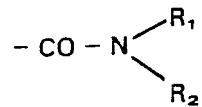 , $-COR_1$, $-COOR_1$, 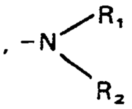 ,

$-NHCOR_1$, 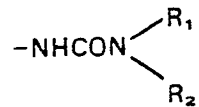 , 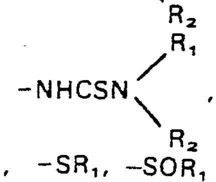 ,

$-NHSO_2R_1$, $-OR_1$, $-OCOR_1$, $-SR_1$, $-SOR_1$ or $-SO_2R_1$,

and, in these formulae, $R_1$ represents a straight-chained or branched alkyl group with 1 to 4 carbon atoms or the phenyl group,

$R_2$ represents a hydrogen atom or an alkyl group with 1 to 4 carbon atoms, and

$R_1$ and $R_2$ may also, together with any nitrogen atom located between them, form a 3- to 6-membered monocyclic, heterocyclic ring, and the physiologically acceptable salts thereof with inorganic or organic bases, characterised in that:

a) a compound of general formula III:

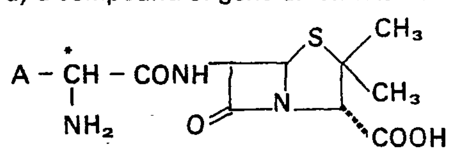

(III)

wherein A is as hereinbefore defined, is reacted with a pyrimidine derivative of general formula IV:

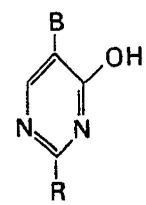

(IV)

wherein:

R is as hereinbefore defined, and

B represents the group –NCO or a reactive derivative of the group –NCOOH, in a solvent at temperatures between –20 and +50°C, or

b) a ureidocarboxylic acid of general formula V:

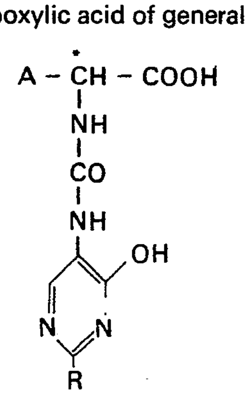

(V)

wherein A and R are as hereinbefore defined, or a salt or reactive derivative thereof, is reacted with 6-aminopenicillanic acid of formula VI:

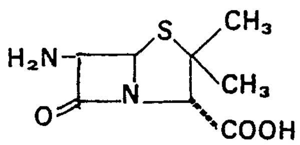

(VI)

or the inorganic or organic salts or reactive derivatives thereof and, if desired, the product thus obtained is subsequently hydrolysed or catalytically hydrogenated to form a penicillin of general formula I or I' and/or, if desired, the penicillin of general formula I or I' thus obtained is subsequently converted into a salt by means of inorganic or organic bases. (Priority of german patent application No. P 2910190.4 of 15.3.1979.)

3. A process as claimed in claims 1a and 2a, characterised in that a pyrimidine derivative of general formula IV as claimed in claims 1a and 2a is used, wherein B represents the group –NCO or wherein B represents a reactive derivative of the group –NHCOOH or, in the latter case, a mixture of such a pyrimidine derivative with another pyrimidine derivative of general formula IV wherein B represents the group –NCO is used, and when an inorganic or organic salt of compound of general formula III is present, the reaction is effected in mixtures of water and water-miscible organic solvents, or in organic solvents, or in mixtures of water and water-immiscible organic solvents in a pH range of between 2.0 and 9.0, or in water as the solvent, in the presence of a base or a buffer solution, or, when a silyl derivative or carboxyl derivative of a compound of general formula III is present, the reaction is effected in anhydrous solvents or solvents free from hydroxyl groups, optionally with the addition of bases.

4. A process as claimed in claims 1b and 2b, characterised in that the reactive derivatives of the ureidocarboxylic acids of general formula V used are the acid anydrides, the reactive esters or amides, the acid halides or acid azides thereof, and the 6-aminopenicillanic acid of formula VI is reacted in the form of its silyl esters, trityl esters, p-nitrobenzyl esters, phenacyl esters and O,N-bis-trimethylsilyl derivatives in an aprotic solvent, but, when used in the form of its salts, it is reacted in a protic solvent, in methylene chloride or in an aqueous medium or in an aqueous organic solvent at temperatures of between –40 and +40°C, optionally in the presence of a b.se and/or a condensing agent, and, if the end product is still present as an ester, the ester group is subsequently split off by hydrolysis or hydrogenolysis.

**Revendications pour les Etats contractants:** BE, CH, DE, FR, GB, IT, LU, NL, SE

1. Nouvelles pénicillines de formule générale I:

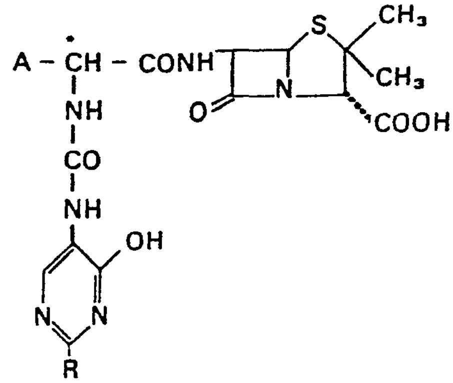

(I)

ou, respectivement, leurs tautomères de formule générale I':

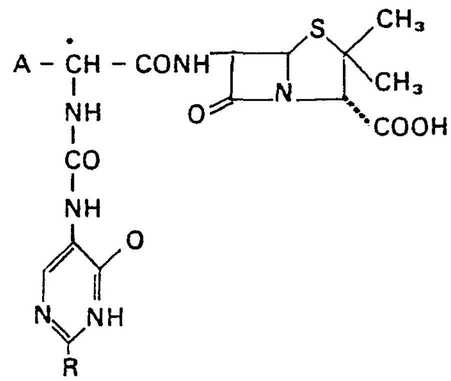

(I')

dans lesquelles:

A représente le groupe phényle, 4-hydroxyphényle ou 2- ou 3-thiényle, ainsi qu'un radical phényle disubstitué en position 3,4, les substituants pouvant être id...tiques ou différents et des atomes de chlore, ..s groupes hydroxy ou méthoxy, et

R représente un groupe de formule générale:

NH–Z–X

dans laquelle: Z représente un groupe alcoylène rectiligne ou ramifié avec 1 à 6 atomes de carbone ou un groupe cycloalcoyle avec 3 à 6 atomes de carbone substitué par le substituant X, et

X représente le groupe cyano, le groupe hydroxy, le groupe mercapto, le groupe aminocarbonyle ou aminosulfonyle ou un groupe selon les formules générales:

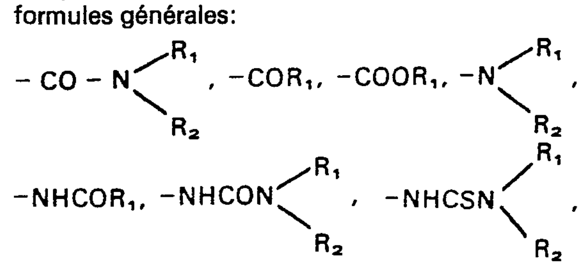

, $-COR_1$, $-COOR_1$, , $-NHCOR_1$, , ,

$-NHSO_2R_1$, $-OR_1$, $-OCOR_1$, $-SR_1$, $-SOR_1$ et $SO_2R_1$, $R_1$ représentant, dans ces formules, un groupe alcoyle rectiligne ou ramifié avec 1 à 4 atomes de carbone ou le groupe phényle, $R_2$ un atome d'hydrogène ou un groupe alcoyle avec 1 à 4 atomes de carbone, et $R_1$ et $R_2$ pouvant également former ensemble avec un atome d'azote éventuellement intermédiaire un cycle hétérocyclique, monocyclique, à 3 à 6 membres,

et leurs sels physiologiquement supportables avec des bases minérales ou organiques.

2. Nouvelles pénicillines de formule générale I:

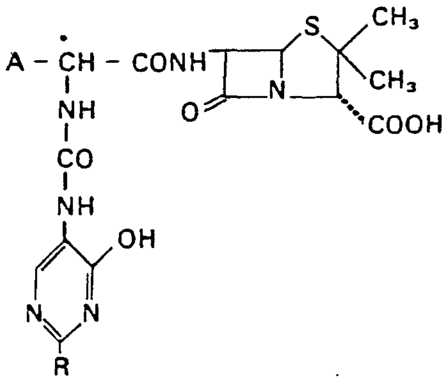

(I)

ou, respectivement, leurs tautomères de formule générale I':

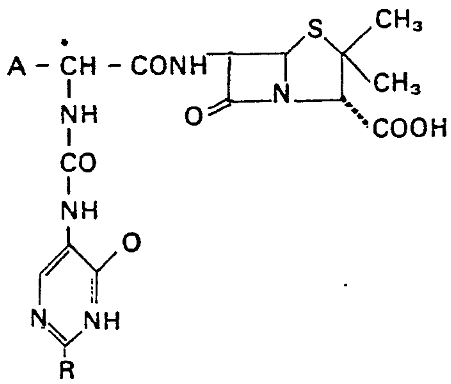

(I')

dans lesquelles A représente le groupe phényle ou p-hydroxyphényle et R un groupe de formule –NH–Z–X, dans cette formule –NH–Z– représentant un groupe correspondant aux formules

$-\underset{H}{\underset{|}{N}}-CH_2-CH_2-$, $-\underset{H}{\underset{|}{N}}(CH_2)_3-$,

$-\underset{H}{\underset{|}{N}}-\underset{CH_3}{\underset{|}{CH}}-CH_2$, $-\underset{H}{\underset{|}{N}}-CH_2-\underset{CH_3}{\underset{|}{CH}}-$

ou $-\underset{H}{\underset{|}{N}}-CH_2-\overset{CH_3}{\overset{|}{\underset{CH_3}{\underset{|}{C}}}}-$

et X signifie le groupe hydroxy, méthoxy, éthoxy, méthoxycarbonyle, éthoxycarbonyle, méthylcarbonyle, éthylcarbonyle, méthylmercapto, éthylmercapto, aminosulfonyle, aminocarbonyle, acétylamino, méthylsulfinyle ou éthylsulfinyle, ou R représente le radical 4'-hydroxycyclohexylamino.

3. Nouvelles pénicillines de formule générale I ou, respectivement, de formule tautomère I' selon les revendications 1 et 2, auxquelles correspond la configuration D=R, et leurs sels physiologiquement supportables avec des bases minérales ou organiques.

4. Procédé pour la préparation des nouvelles pénicillines de formule générale I:

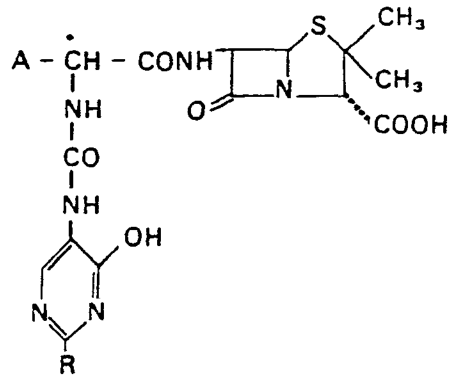

(I)

ou, respectivement, des tautomères de formule générale I':

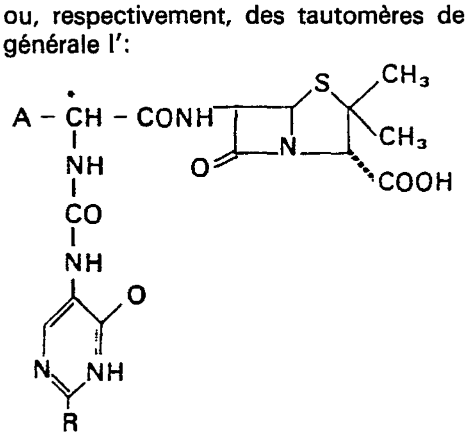

(I')

dans lesquelles A signifie le groupe phényle, 4-hydroxyphényle ou 2- ou 3-thiényle, ainsi qu'un radical phényle disubstitué en position 3,4, les substituants pouvant être identiques ou différents et des atomes de chlore, des groupes hydroxy ou méthoxy, et

R signifie un groupe de formule générale:

NH–Z–X,

dans laquelle:

Z représente un groupe alcoylène rectiligne ou ramifié avec 1 à 6 atomes de carbone ou un groupe cycloalcoyle avec 3 à 6 atomes de carbone substitué par le substituant X, et

X représente le groupe cyano, le groupe hydroxy, le groupe mercapto, le groupe aminocarbonyle ou aminosulfonyle ou un groupe correspondant aux formules générales:

$-CO-N\begin{smallmatrix}R_1\\R_2\end{smallmatrix}$, $-COR_1$, $-COOR_1$, $-N\begin{smallmatrix}R_1\\R_2\end{smallmatrix}$,

$-NHCOR_1$, $-NHCON\begin{smallmatrix}R_1\\R_2\end{smallmatrix}$, $-NHCSN\begin{smallmatrix}R_1\\R_2\end{smallmatrix}$,

$-NHSO_2R_1$, $-OR_1$, $-OCOR_1$, $-SR_1$, $-SOR_1$ et $-SO_2R_1$, $R_1$ représentant, dans ces formules, un groupe alcoyle rectiligne ou ramifié avec 1 à 4 atomes de carbone ou le groupe phényle, $R_2$ un atome d'hydrogène ou un groupe alcoyle avec 1 à 4 atomes de carbone, et $R_1$ et $R_2$ pouvant également former ensemble avec un atome d'azote éventuellement intermédiaire un cycle hétérocyclique, monocyclique à 3 à 6 membres,

et de leurs sels physiologiquement supportables avec des bases minérales ou organiques, caractérisé en ce que:

a) on fait réagir un composé de formule générale III:

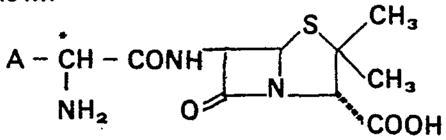

(III)

dans laquelle A est défini comme plus haut, avec un dérivé de pyrimidine de formule générale IV:

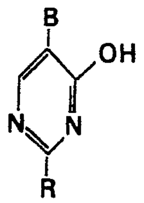

(IV)

dans laquelle R est défini comme plus haut et B signifie le groupe –NCO ou un dérivé réactif du groupe –NHCOOH,

dans un solvant à des températures comprises entre –20 et +50° C, ou

b) on fait réagir un acide uréidocarboxylique de formule générale V:

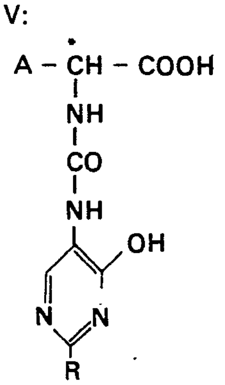

(V)

dans laquelle:

A et R possèdent les significations indiquées plus haut, ou, respectivement, leurs sels ou dérivés réactifs avec l'acide 6-aminopénicillanique de formule VI:

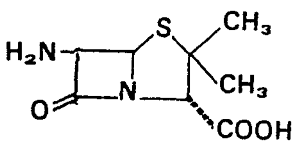

(VI)

ou ses sels minéraux ou organiques ou dérivés réactifs et, éventuellement ensuite, on hydrolyse ou hydrogénolyse catalytiquement le produit ainsi obtenu en une pénicilline de formule générale I ou, respectivement, I' et/ou, éventuellement ensuite, on transforme en son sel la pénicilline obtenue de formule générale I ou, respectivement, I' au moyen de bases minérales ou organiques.

5. Procédé selon la revendication 4a, caractérisé en ce qu'on utilise un dérivé de pyrimidine de formule générale IV selon la revendication 4a, dans laquelle B signifie le groupe –NCO ou dans laquelle B signifie un dérivé réactif du groupe –NHCOOH ou, dans ce dernier cas, un mélange d'un tel dérivé de pyrimidine avec un autre dérivé de pyrimidine de formule générale IV dans laquelle B représente le groupe –NCO, et on effectue la réaction soit en présence d'un sel minéral ou organique d'un composé de formule générale III, dans des mélanges d'eau avec des solvants organiques miscibles à l'eau ou, respectivement, dans des solvants organiques ou, respectivement, dans des mélanges d'eau et de solvants organiques non miscibles à l'eau dans une gamme de pH comprise entre 2,0 et 9,0 ou, respectivement, dans l'eau comme solvant en présence d'une base, ou, respectivement, d'une solution tampon ou, en présence d'un dérivé silylé ou d'un dérivé carboxylé, d'un composé de formule générale III, dans des solvants anhydres et ne comprenant pas de groupes hydroxy, éventuellement avec addition de bases.

6. Procédé selon la revendication 4b, caractérisé en ce qu'on utilise, en tant que dérivés réactifs, des acides uréidocarboxyliques de formule générale V, leurs anhydrides d'acides, leurs esters ou amides réactifs, leurs halogénures d'acides ou azides d'acides, et on fait réagir l'acide 6-aminopénicillanique de formule VI sous la forme de ses esters silylé, tritylé, p-nitrobenzylé, phénacylé et O,N-bis-triméthylsilylé dans un solvant aprotique, cependant sous la forme de ses sels dans un solvant protique, dans le chlorure de méthylène ou dans un milieu aqueux ou dans un solvant hydro-organique, à des températures comprises entre –40 et +40° C, éventuellement en présence d'une base et/ou d'un agent de condensation et, si le produit final se présente encore à l'état d'ester, on hydrolyse ou hydrogénolyse ensuite le groupe ester.

7. Médicament contenant un ou plusieurs composés selon l'une des revendications 1 ou 2, conjointement aux excipients et/ou adjuvants habituels.

8. Médicament contenant au moins un composé selon l'une des revendications 1 ou 2, conjointement à un antibiotique aminoglycosidique ou son sel pharmacologiquement supportable et à un excipient et/ou adjuvant habituel.

**Revendications pour l'Etat contractant:** AT

1. Procédé pour la préparation de nouvelles pénicillines de formule générale I:

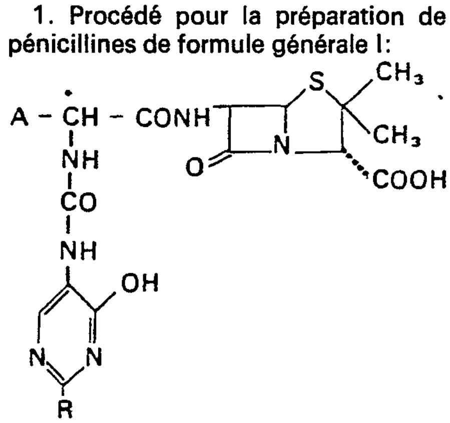

(I)

ou, respectivement, des tautomères de formule générale I':

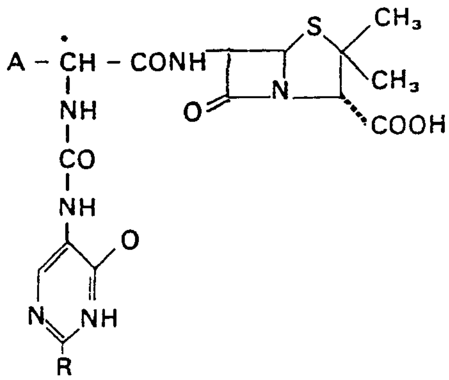

(I')

dans lesquelles:

A représente le groupe phényle, 4-hydroxyphényle ou 2- ou 3-thiényle, ainsi qu'un radical phényle disubstitué en position 3,4, les substituants pouvant être identiques ou différents et des atomes de chlore, des groupes hydroxy ou méthoxy, et

R représente un groupe de formule générale:

NH–Z–X

dans laquelle:

Z représente un groupe alcoylène rectiligne ou ramifié avec 1 à 6 atomes de carbone ou un groupe cycloalcoyle avec 3 à 6 atomes de carbone, substitué par le substituant X, et

X représente le groupe cyano, le groupe hydroxy, le groupe mercapto, le groupe aminocarbonyle ou aminosulfonyle ou un groupe selon les formules générales:

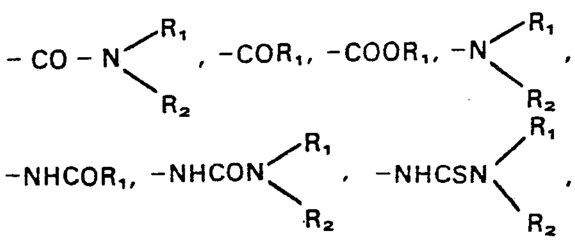

$-NHSO_2R_1$, $-OR_1$, $-OCOR_1$, $-SR_1$, $-SOR_1$ et $SO_2R_1$, $R_1$ représentant, dans ces formules, un groupe alcoyle rectiligne ou ramifié avec 1 à 4 atomes de carbone ou le groupe phényle, $R_2$ un atome d'hydrogène ou un groupe alcoyle avec 1 à 4 atomes de carbone, et $R_1$ et $R_2$ pouvant également former ensemble avec un atome d'azote éventuellement intermédiaire un cycle hétérocyclique, monocyclique, à 3 à 6 membres,

et de leurs sels physiologiquement supportables avec des bases minérales ou organiques, caractérisé en ce que:

a) on fait réagir un composé de formule générale III:

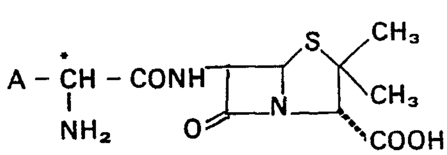

(III)

dans laquelle A est défini comme plus haut, avec un dérivé de pyrimidine de formule générale IV:

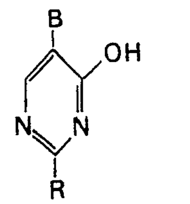

(IV)

dans laquelle R est défini comme plus haut et B signifie le groupe –NCO ou un dérivé réactif du groupe –NHCOOH,

dans un solvant à des températures comprises entre –20 et +50° C, ou

b) on fait réagir un acide uréidocarboxylique de formule générale V:

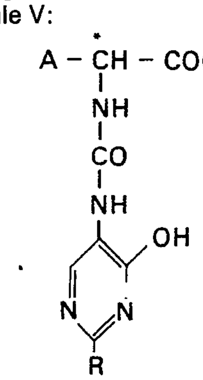

(V)

dans laquelle:

A et R possèdent les significations indiquées plus haut, ou, respectivement, leurs sels ou dérivés réactifs avec l'acide 6-aminopénicillanique de formule VI:

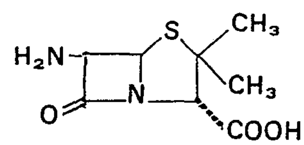

(VI)

ou ses sels minéraux ou organiques ou dérivés réactifs et, éventuellement ensuite, on hydrolyse ou hydrogénolyse catalytiquement le produit ainsi obtenu en une pénicilline de formule générale I ou, respectivement, I' et/ou éventuellement ensuite on transforme en son sel la pénicilline obtenue de formule générale I ou, respectivement, I' au moyen de bases minérales ou organiques.

2. Procédé pour la préparation de nouvelles pénicillines de formule générale I:

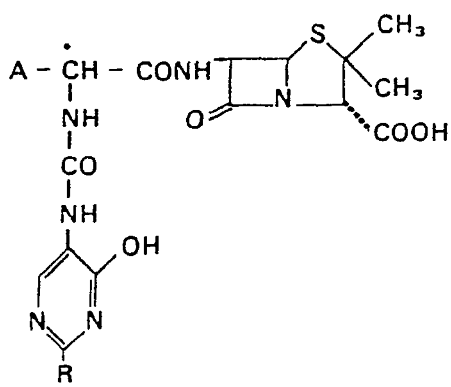

(I)

ou, respectivement, de leurs tautomères de formule générale I':

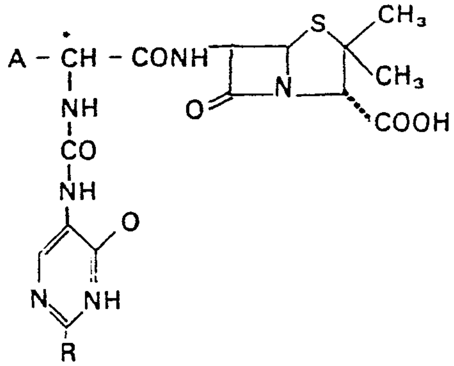

(I')

dans lesquelles A représente le groupe phényle, 4-hydroxyphényle ou 2- ou 3-thiényle, ainsi qu'un radical phényle disubstitué en position 3,4, les substituants pouvant être identiques ou différents et des atomes de chlore, des groupes hydroxy ou méthoxy, et

R représente un groupe de formule générale:

NH–Z–X

dans laquelle:

Z représente un groupe alcoylène rectiligne ou ramifié avec 1 à 6 atomes de carbone ou un groupe cycloalcoyle avec 3 à 6 atomes de carbone substitué par le substituant X, et

X représente le groupe cyano, le groupe hydroxy, le groupe mercapto ou un groupe correspondant aux formules générales:

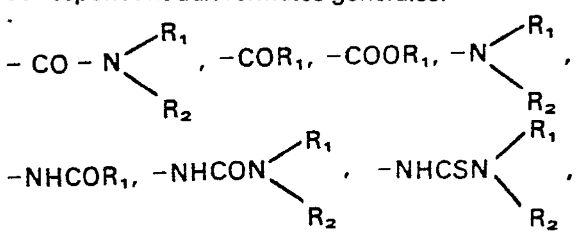

$-NHSO_2R_1$, $-OR_1$, $-OCOR_1$, $-SR_1$, $-SOR_1$, et $SO_2R_1$, $R_1$ représentant, dans ces formules, un groupe alcoyle rectiligne ou ramifié avec 1 à 4 atomes de carbone ou le groupe phényle, $R_2$ un atome d'hydrogène ou un groupe alcoyle avec 1 à 4 atomes de carbone, et $R_1$ et $R_2$ pouvant également former ensemble avec un atome d'azote éventuellement intermédiaire un cycle hétérocyclique, monocyclique à 3 à 6 membres,

et de leurs sels physiologiquement supportables avec des bases minérales ou organiques, caractérisé en ce que:

a) on fait réagir un composé de formule générale III:

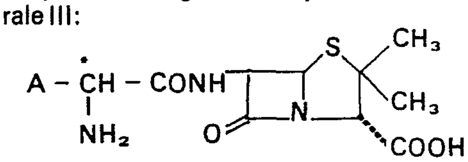

(III)

dans laquelle A est défini comme plus haut, avec un dérivé de pyrimidine de formule générale IV:

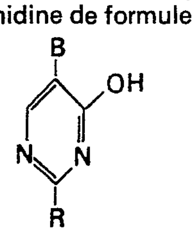

(IV)

dans laquelle R est défini comme plus haut et B signifie le groupe –NCO ou un dérivé réactif du groupe –NHCOOH

dans un solvant à des températures comprises entre –20 et +50°C, ou

b) on fait réagir un acide uréidocarboxylique de formule générale V:

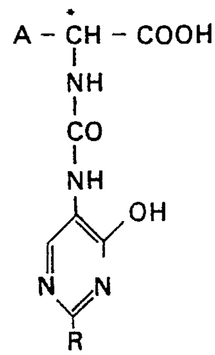

(V)

dans laquelle:

A et R possèdent les significations indiquées plus haut, ou, respectivement, leurs sels ou dérivés réactifs avec l'acide 6-aminopénicillanique de formule VI:

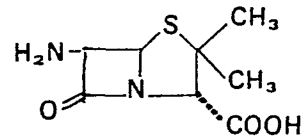

(VI)

ou ses sels minéraux ou organiques ou dérivés réactifs et, éventuellement ensuite, on hydrolyse ou hydrogénolyse catalytiquement le produit ainsi obtenu en une pénicilline de formule générale I ou, respectivement, I' et/ou, éventuellement ensuite, on transforme en son sel la pénicilline obtenue de formule générale I ou, respectivement, I' au moyen de bases minérales ou organiques. (Priorité de la demande de brevet allemand N° P 2910190.4 du 15.3.1979.)

3. Procédé selon les revendications 1a et 2a, caractérisé en ce qu'on utilise un dérivé de pyrimidine de formule générale IV selon les revendications 1a et 2a, dans laquelle B signifie le groupe –NCO ou dans laquelle B signifie un dérivé réactif du groupe –NHCOOH ou, dans ce dernier cas, un mélange d'un tel dérivé de pyrimidine avec un autre dérivé de pyrimidine de formule générale IV, dans laquelle B représente le groupe –NCO, et on effectue la réaction soit, en présence d'un sel minéral ou organique d'un composé de formule générale III, dans des mélanges d'eau avec des solvants organiques miscibles à l'eau ou, respectivement, dans des solvants organiques ou, respectivement, dans des mélanges d'eau et de solvants organiques non miscibles à l'eau dans une gamme de pH comprise entre 2,0 et 9,0 ou, respectivement, dans l'eau comme solvant en présence d'une base ou, respectivement, d'une solution tampon, ou, en présence d'un dérivé silylé ou d'un dérivé carboxylé, d'un composé de formule générale III, dans des solvants anhydres et ne comprenant pas de groupes hydroxy, éventuellement avec addition de bases.

4. Procédé selon les revendications 1b et 2b, caractérisé en ce qu'on utilise, en tant que dérivés réactifs, des acides uréidocarboxyliques de formule générale V, leurs anhydrides d'acides, leurs esters ou amides réactifs, leurs halogénures d'acides ou azides d'acides, et on fait réagir l'acide 6-aminopénicillanique de formule VI sous la forme de ses esters silylé, tritylé, p-nitrobenzylé, phénacylé et O,N-bis-triméthylsilylé dans un solvant aprotique, cependant sous la forme de ses sels dans un solvant protique, dans la chlorure de méthylène ou dans un milieu aqueux ou dans un solvant hydro-organique, à des températures comprises entre –40 et +40° C, éventuellement en présence d'une base et/ou d'un agent de condensation et, si le produit final se présente encore à l'état d'ester, on hydrolyse ou hydrogénolyse ensuite le groupe ester.